# EUROPEAN PATENT SPECIFICATION

(11) **EP 3 334 836 B1**
(45) Date of publication and mention of the grant of the patent: **15.04.2020**
(21) Application number: 16750751.6
(22) Date of filing: 04.08.2016
(51) Int. Cl.: C12Q 1/68

(54) **METHOD FOR PREDICTING RADIOSENSITIVITY OF A CELL**
VERFAHREN ZUR VORHERSAGE DER STRAHLENSENSIBILISIERUNG EINER ZELLE
PROCÉDÉ PERMETTANT DE PRÉDIRE LA RADIOSENSIBILITÉ D'UNE CELLULE

(30) Priority: 13.08.2015 EP 15002426
(43) Date of publication of application: 20.06.2018
(73) Proprietor: Universität Duisburg-Essen, 45141 Essen (DE)
(72) Inventor: ILIAKIS, George, 40489 Düsseldorf (DE)
(74) Representative: Lahrtz, Fritz
(86) International application number: PCT/EP2016/068668
(87) International publication number: WO 2017/025436

(56) References cited:
- WO-A1-2015/042570
- YANLEI CHENG ET AL: "The yield of DNA double strand breaks determined after exclusion of those forming from heat-labile lesions predicts tumor cell radiosensitivity to killing", RADIOTHERAPY AND ONCOLOGY, vol. 116, no. 3, 1 September 2015 (2015-09-01), pages 366-373, XP055248301, Ireland ISSN: 0167-8140, DOI: 10.1016/j.radonc.2015.08.004
- SATYENDRA K SINGH ET AL: "Reduced contribution of thermally labile sugar lesions to DNA double strand break formation after exposure to heavy ions", RADIATION ONCOLOGY, BIOMED CENTRAL LTD, LO, vol. 8, no. 1, 2 April 2013 (2013-04-02), page 77, XP021145740, ISSN: 1748-717X, DOI: 10.1186/1748-717X-8-77
- M. GULSTON: "Processing of clustered DNA damage generates additional double-strand breaks in mammalian cells post-irradiation", NUCLEIC ACIDS SYMPOSIUM SERIES, vol. 32, no. 4, 23 February 2004 (2004-02-23), pages 1602-1609, XP055248295, GB ISSN: 0261-3166, DOI: 10.1093/nar/gkh306

## Description

Optimal design of radiotherapy regimens should consider the radiosensitivity of normal as well as of tumor tissue, for each patient individually. Estimates of these radiosensitivity parameters can be obtained by *in-vitro* analysis of fibroblast (1-9) and tumor cell cultures (10-12), respectively, generated from each cancer patient (4). Related studies have indeed shown pronounced differences of fibroblast radiosensitivity in cultures generated from different individuals (1-9). Notably, in some of these studies a good correlation between fibroblast radiosensitivity *in-vitro* and normal tissue reaction during therapy *in-vivo* could be demonstrated (4, 13-16). A similar correlation between *in-vitro* radiosensitivity and *in-vivo* response has been shown for some tumors and their derivative cell cultures (10-12). Collectively, these studies suggest that radiosensitivity information can decisively contribute to the design of improved radiotherapy regimens.

The standard of radiosensitivity determination is colony formation. However, the approach is laborious as it requires generation from each patient of fibroblast and tumor cell cultures, a particularly demanding task, which even when successful takes several weeks. Notably, the outcome of such efforts strongly depends on experimental conditions and the on-site available technical skills, limiting its applicability to highly specialized clinical centers. Therefore, surrogate parameters enabling faster and easier prediction of *in-vitro* radiosensitivity to killing are urgently needed and indeed, in several tertiary clinical centers actively pursued.

Killing of cells exposed to ionizing radiation (IR) is widely attributed to the formation of chromosomal abnormalities from error-prone processing of DNA double- strand breaks (DSBs) (17). Therefore, induction and repair of DSBs have been extensively investigated as surrogate-parameters of cell radiosensitivity to killing. Despite initial claims (11, 18, 19), induction of DSBs as measured by neutral filter elution (20, 21), or pulsed-field gel electrophoresis using standard lysis protocols (9, 12), rarely shows a robust correlation with cell radiosensitivity to killing (19, 22). On the other hand, a correlation between cellular radiosensitivity and residual DSBs has been documented (5-8).

Residual DSBs as surrogate of cell radiosensitivity to killing has two limitations. First, its reliable determination requires high radiation doses, and second, the parameter fails to consider lethal events manifesting as chromosome translocations. Translocations reflect DSB rejoining and are therefore associated with the elimination of DSBs from the initial pool, despite the fact that they contribute at the same time markedly to cell lethality and thus to radiosensitivity to killing.

More recently, γ-H2AX foci formation and decay have been explored as a surrogate of cell radiosensitivity to killing (23-26). This parameter has the distinct advantage that it can be determined at relatively low doses of radiation, but has other limitations and shortcomings (27).

Thus, despite substantial efforts, generally applicable and validated surrogate assays for cell radiosensitivity to killing are lacking and the field remains in its infancy, despite the urgent need and the great potential of this kind of information in the improvement of radiation oncology.

There is, thus, a need in the art for methods allowing the prediction of the degree of radiosensitivity of a cell in a generally applicable and reliable manner. This problem is solved by the present invention by the provision of the claims.

All studies carried out thus far to correlate induction of DSBs with cell radiosensitivity to killing assume that the methods employed detect DSBs actually present in cells immediately after radiation exposure. However, it is now known that ionizing radiation (IR) induces, in addition to sugar lesions promptly disrupting the sugar-phosphate backbone (prompt breaks) to form DSBs (prDSBs), also lesions doing so after temperature-dependent chemical processing (delayed breaks) (28-31). These thermally labile sugar lesions, TLSLs, constitute what are considered radiation-induced labile sites.

Chemical evolution of TLSLs to single-strand breaks (SSBs) generates additional, TLSL-dependent DSBs (tIDSBs). These delayed-forming DSBs are thought to form continuously within cells during the first postirradiation hour and to add to prDSBs (29). Importantly, TLSLs invariably convert to breaks when lysis is carried out at elevated temperatures (above 20°C), and as a result most measurements of DSB induction relying on physical analysis of DNA size reflect the sum of prDSBs + tIDSBs (to be referred to here as total DSBs, tDSBs) (29, 32, 33). Incidentally, the same holds true for γ-H₂AX based analysis of DSB induction and processing (29).

This new information on DSB nature is used herein to address the correlation between induction of DSBs and cell radiosensitivity to killing. Thereby, a surprisingly good correlation between the yield of prDSBs and the degree of radiosensitivity has been found in a very large series of human cell lines.

In an aspect, the present invention provides an in vitro method for predicting the degree of radiosensitivity of a cell, comprising
(a) irradiating a cell,
(b) determining the number of prompt double-strand breaks (prDSBs) in the cell of step (a), and
(c) using the number of prDSBs determined in step (b) to predict the degree of radiosensitivity of said cell.

In another aspect, the present invention provides an in vitro method for predicting the degree of radiosensitivity of a cell, comprising
(a) irradiating a cell,
(b) determining the number of thermally labile sugar lesion-dependent double-strand breaks (tIDSBs)) in the cell of step (a), and
(c) using the number of tIDSBs determined in step (b) to predict the degree of radiosensitivity of said cell.

Preferably, the number of tIDSBs is determined by subtracting the number of prDSBs from the number of total double-strand breaks (tDSBs).

In an embodiment of the aspects of the invention referred to above, the cell is a diseased cell, preferably a tumor cell, more preferably of epithelial origin, of mesenchymal origin, of hematopoietic origin, or of neuro-ectodermal origin. Yet more preferred, the cell is selected from a breast adenocarcinoma, sweat gland adenocarcinoma, salivary gland adenocarcinoma, skin squamous cell carcinoma, adenocarcinoma of the thyroid, lung, stomach, liver, pancreas, small intestine, colon, or prostate, transitional cell carcinoma of the bladder; adenocarcinoma of the kidney, testis or endometrium, fibrosarcoma, liposarcoma, osteosarcoma, chondrosarcoma, leiomyosarcoma, hemangiosarcoma, lymphoma, leukemia, astrocytoma, retinoblastoma, oligodendroglioma, schwannoma, melanoma, head and neck cancer, ovarian cancer, adenoid carcinoma, basal cell carcinoma, epidermoid carcinoma, meningioma, neurofibroma, glioblastoma, ependymoma, medulloblastoma, neuroblastoma, hepatoma, mesothelioma, brain cancer such as glioblastoma multiforme, hepatoma, lymphoma, myeloma, neuroblastoma, sarcoma, stomach cancer, thyroid cancer, non-melanoma skin cancer, non-small cell lung cancer, cervical cancer, or anal cancer. Or the diseased cell is preferably from the thyroid gland in case of the disease of Basedow or hyperthyroidism, from the pituitary gland in case of pituitary adenoma, from the meninges in case of a meningioma, from the skin with a non-cancerous skin disorder, particularly rosacea, poikiloderma of Civatte, angioma, telangiectasias, or psoriasis, or from the ankle in case of talalgia.

In a further embodiment, the degree of radiosensitivity of said cell is predicted with respect to a reference.

Preferably, the reference is a diseased individual, diseased tissue or diseased cell or a plurality of diseased individuals, diseased tissues or diseased cells, wherein the disease may be a tumor or disease as defined above, more preferably the reference is a tumor cell or a plurality of tumor cells. The disease such as the tumor is preferably known to be treatable by radiotherapy. The radiation dose for treating the disease of the reference such as the tumor is also preferably known.

Such reference may, on the other hand, be a normal individual, normal tissue or normal cell or a plurality of normal individuals, normal tissues or normal cells.

The present invention furthermore provides the above methods for determining the radiation dose for treating a diseased cell, preferably a tumor cell, in an individual, the method further comprising in addition to items (a) to (c)
d) comparing the radiosensitivity of the diseased cell, preferably the tumor cell, with the radiosensitivity of the reference, and
e) determining the radiation dose for optimally treating the diseased cell, preferably the tumor cell.

In a further embodiment of the above aspects of the invention, the cell is a normal cell, preferably an epithelial cell, such as a keratinocyte or a lens epithelial cell, a melanocyte, a cardiac myocyte, a chrondrocyte, an endothelial cell, a fibroblast, an osteoblast, a preadipocyte, a skeletal muscle cell, a smooth muscle cell, or a lymphocyte.

Also in the case, where the cell is a normal cell, the degree of radiosensitivity of said cell may be predicted with respect to a reference. Such reference may be a normal individual, normal tissue or normal cell or a plurality of normal individuals, normal tissues or normal cells.

In a further embodiment of the methods referred to above, the cell is irradiated with ionizing radiation.

In a still further embodiment of the methods referred to above, the number of prDSBs or tIDSBs is determined using pulsed-field gel electrophoresis, preferably asymmetric field inversion gel electrophoresis, or the "Comet" assay.

In a still further embodiment of the methods referred to above, the number of prDSBs or tIDSBs is determined by the fraction of DNA released (FDR). Preferably, the FDR is the fraction of DNA released from the well containing the cells in the case of pulsed-field gel electrophoresis, or from the cell nucleus in the case of the "Comet" assay.

In the following, the present invention is described in detail. The features of the present invention are described in individual paragraphs. This, however, does not mean that a feature described in a paragraph stands isolated from a feature or features described in other paragraphs. Rather, a feature described in a paragraph can be combined with a feature or features described in other paragraphs. For example, if in a paragraph the method for determining the number of prDSBs or tIDSBs is described and in another paragraph the cell to be used in the method of the present invention is described, then it is clear that the method of the present invention includes the combination of the disclosed determination methods with the described types of cells.

The term "comprising" as used herein is meant to "include or encompass" the disclosed features and further features which are not specifically mentioned. The term "comprising" is also meant in the sense of "consisting of" the indicated features, thus not including further features except the indicated features. Thus, the method of the present invention may be characterized by additional features in addition to the features as indicated.

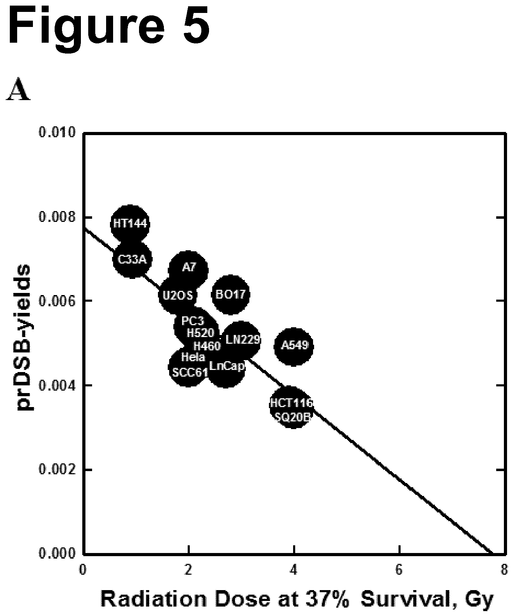

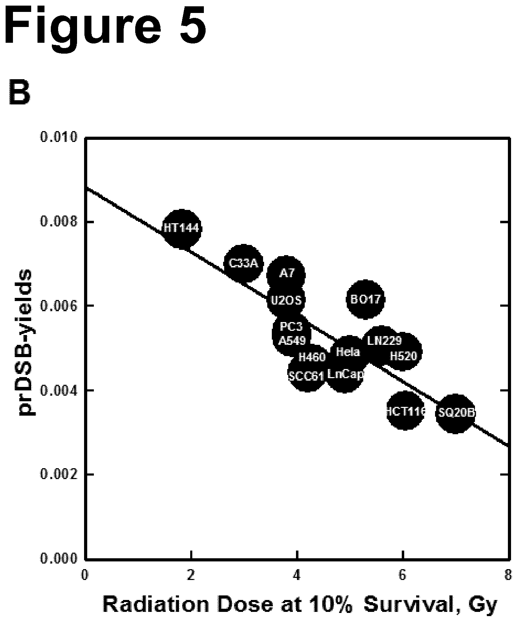

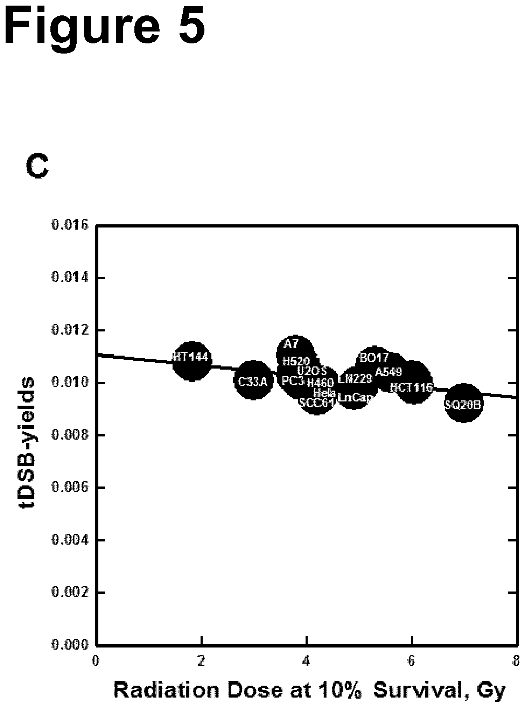

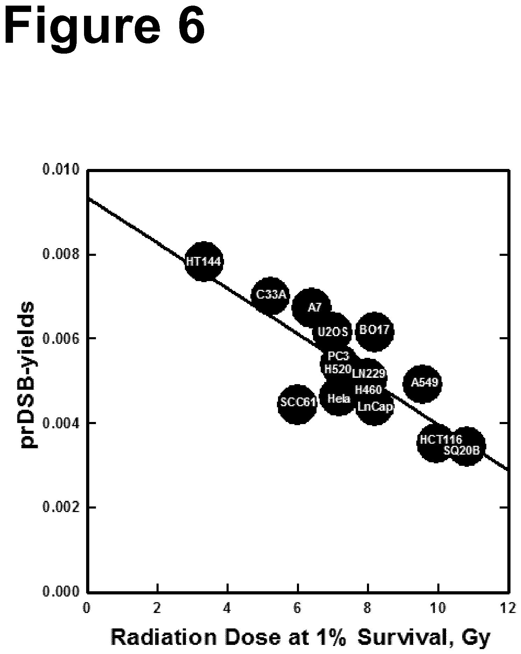

The present invention relates to a predictive method for determining the degree of radiosensitivity of a cell. The present inventors have found a high correlation between the number of prDSBs in the DNA of a cell, which are generated due to irradiation with ionizing radiation, and the degree of radiosensitivity of the cell. Figures 5 and 6 show that the different cell lines tested generate different numbers of prDSBs/Gray upon irradiation. Cell lines which generate higher numbers of prDSBs/Gray are already killed by a relatively low radiation dose, while cell lines which generate lower numbers of prDSBs/Gray need higher radiation doses for killing. For example, the cell line HT144 which shows a low survival even at relatively low doses of irradiation correlates with a high number of prDSBs/Gray. In contrast thereto, the cell line SQ20B, which needs relatively high radiation doses for killing, shows a relatively low number of prDSBs/Gray. Thus, the higher radiosensitivity of the cell line HT144 versus the cell line SQ20B is truly reflected by the number of prDSBs/Gray.

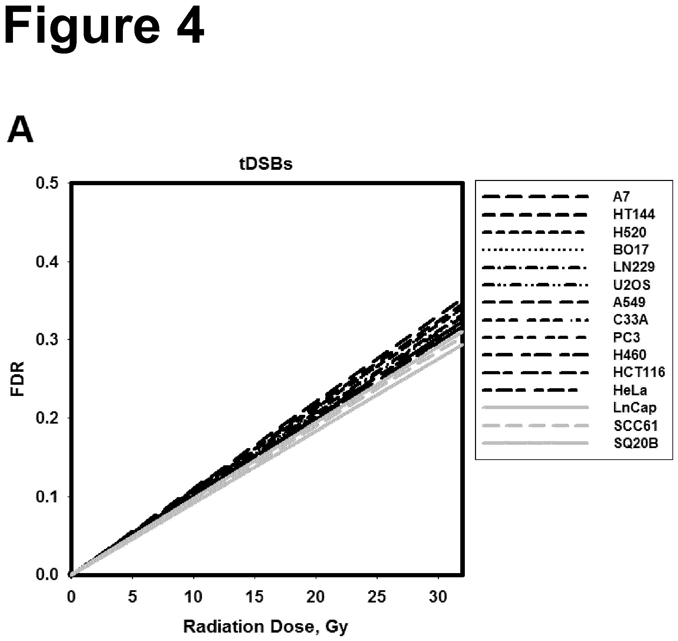

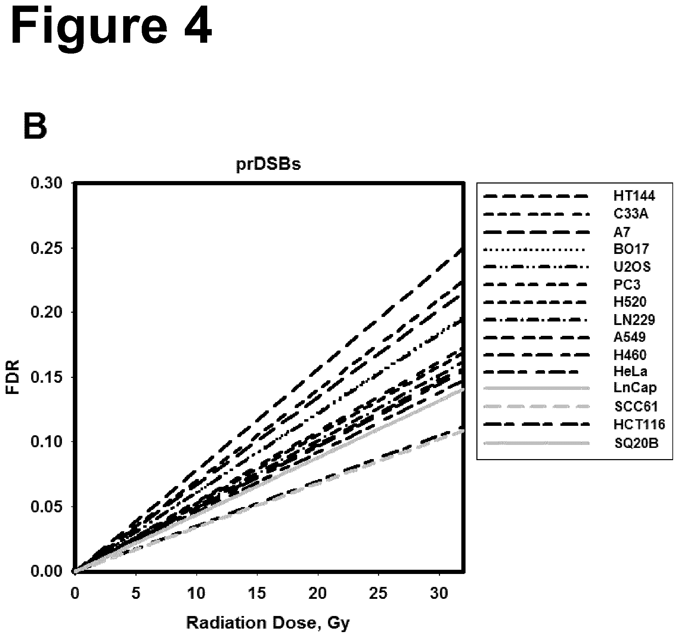

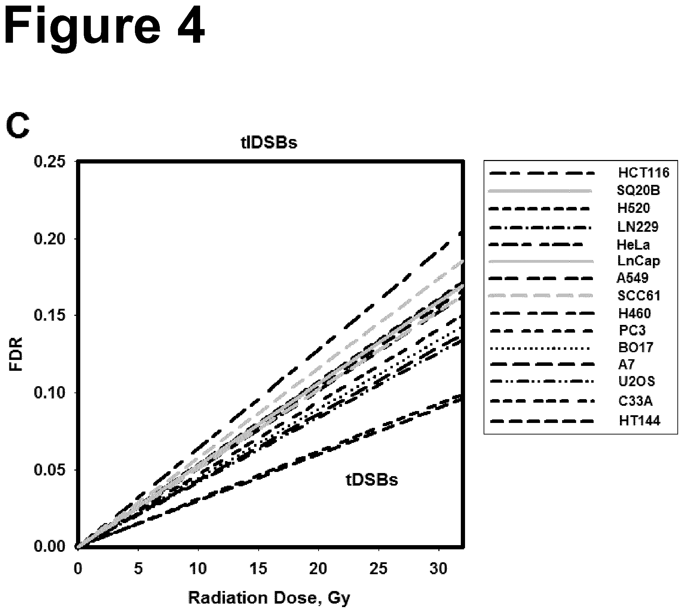

Moreover, the present inventors have found that tIDSBs are predictive of the degree of radiosensitivity of a cell. Figure 4 shows a high similarity of induction of tIDSBs and a marked difference of induction of prDSBs between the different cell lines tested. Therefore, the resulting marked differences of induction of tIDSBs between the different cell lines tested, calculated by subtracting the number of prDSBs from the number of tDSBs, are predictive of the degree of radiosensitivity of a cell.

It was previously shown that in cells which have been exposed to ionizing radiation two kinds of DNA double strand breaks exist, prompt double strand breaks (prDSBs) and thermo-labile sugar lesion-dependent double strand breaks (tIDSBs). While it has been possible in the art to separate and quantitatively determine prDSBs and tIDSBs, it has not been known in the art that the measurement of prDSBs enables the determination of the degree of radiosensitivity of a cell with an accuracy which has never been achieved by using previous methods. Based on this finding, the present inventors have developed a method which enables the skilled person to predict radiosensitivity of a cell with high accuracy.

The mechanism by which irradiation of a cell results in a harmful effect, predominantly killing, involves molecular damage, in particular of DNA, leading to the formation of DNA double strand breaks (DSBs). DSBs are considered responsible for the harmful effects of radiation, such as cell killing, since they interfere with normal cell proliferation and the process of mitosis. IR induces DSBs by first inducing sugar lesions in DNA molecules, some of which promptly disrupt the sugar-phosphate backbone and result in prompt double strand breaks (prDSBs) of the DNA. These prompt breaks occur as an immediate effect of the ionizing radiation on DNA molecules. IR also induces sugar lesions that fail to promptly disrupt the sugar-phosphate backbone of the DNA and to cause a prDSB, do so however at later times as a result of thermal evolution and cause DSBs. Sugar lesions with this property are named thermally labile sugar lesions (TLSLs), and the DSBs generated by their temperature-dependent chemical processing, TLSL-dependent DSBs (tIDSBs). These delayed-forming DSBs are thought to be generated within cells during the first post-irradiation hour.

As used herein, the term "prDSBs" or "prompt double strand breaks" means DNA double strand breaks which occur immediately after irradiation with IR as a direct effect of irradiation. They evolve from sugar lesions which are induced by IR and which promptly disrupt the DNA. As used herein, the term "tIDSBs" or "thermally labile sugar lesion-dependent double strand breaks" means DNA double strand breaks which occur as delayed breaks chemically evolving from thermally labile sugar lesions, dependent on temperature. The thermally labile sugar lesions induced by IR chemically evolve as a function of temperature. The temperature required for such chemical evolution, and thus also the generation of tIDSBs, typically lies above 20°C. As used herein, the term "tDSBs" or "total double strand breaks" means the totality of DNA double strand breaks which are induced by IR. They are the sum of prDSBs and tIDSBs.

People differ by their degree of radiosensitivity. In seemingly healthy people, there is a wide range of radiosensitivities. But also cells within an individual differ by their degree of radiosensitivity. Also tumor cells differ widely with respect to their degree of radiosensitivity, dependent on the kind of tumor and the genetic changes associated with its induction. Moreover, tumor cells can also differ with respect to their degree of radiosensitivity from normal cells which are derived from the same tissue. The determination of the differences of radiosensitivities of cells within and between individuals is a necessary requirement for an effective protection against radiation, or for the design of effective radiotherapy.

Radiation protection is the science and practice of protecting people and the environment from the harmful effects of ionizing radiation. Ionizing radiation is widely used in industry and medicine and can represent a significant health hazard. Fundamental to radiation protection is the reduction of radiation dose an individual is exposed to, e.g. by wearing protective clothing, and the accurate determination of radiation dose received by an individual. Radiation protection can be optimized by determining the radiosensitivity of an individual and use it as basis for prediction and control of the risk associated with a radiation exposure in clinical and occupational settings. For example, knowledge of the degree of radiosensitivity of a person may help to decide whether the person is suitable for employment in nuclear power reactors, or other scientific and clinical fields utilizing ionizing radiation as a tool. It can also be used to decide whether a person should be preferentially removed from a risk area, e.g., an area of radiation accident, if this person is particularly radiosensitive.

Radiotherapy or radiation therapy is a form of cancer therapy, which utilizes ionizing radiation for eradicating diseased or malignant cells. Typically, radiotherapy is a component of a tumor or cancer management scheme designed to control tumor growth by killing and/or impairing the growth of malignant cells. Since the radiosensitivity of malignant cells varies widely among different forms of cancer, among individuals for the same form of cancer, and occasionally for the same individual in the course of therapy, ideally, the practitioner should know the current degree of radiosensitivity of each individual tumor under treatment in order to be able to decide whether radiotherapy is a suitable treatment form, and if yes to estimate the radiation dose required for achieving local control through the killing of tumor cells. While some tumor types may be treated by relatively low radiation doses, others may need high radiation doses in order to achieve similar cell killing of tumor cells and thus overall similar tumor control. However, if a particular tumor requires high doses of radiation for effective tumor control, a practitioner may prefer to select another treatment form in an effort to minimize side effects arising from unavoidable irradiation of normal tissues. Alternatively, a practitioner may develop special treatment planning protocols or opt for types of radiation that minimize such side effects. Such decisions can be reached rationally if the radiosensitivities of tumor cells under treatment are estimated and compared to a reference. The decision regarding how to treat a tumor by radiotherapy would then be made differently for tumors which prove to have a higher degree of radiosensitivity as compared to such reference than for tumors that are radioresistant in comparison to the same reference. In extreme cases of tumor radioresistance, the practitioner may even opt for another form of treatment all together for the benefit of the patient.

Moreover, the degree of radiosensitivity of a tumor to be treated will determine the radiation dose to be used for treatment. At present radiation dose determinations for the treatment of a particular type of cancer are empirical and do not consider radiosensitivity differences that are known to exist among tumor cells arising in different individuals. In view of the above, it becomes evident that knowledge of the degree of radiosensitivity of a tumor cell as compared to the radiosensitivity of another cell, such as a reference tumor cell, may help significantly, first in the selection of radiotherapy as the suitable treatment modality, and subsequently in the determination of the radiation dose required to achieve effective local control of tumor growth. Furthermore, knowledge of the degree of radiosensitivity, not only of the tumor cells but also of the adjacent normal cells may provide further information useful in the design of treatment planning protocols achieving optimal tumor growth control, while ensuring at the same time minimal radiation damage to adjacent normal tissues.

The method of the present invention is in principle applicable for the determination of the radiosensitivity to killing of any diseased or normal cell. This information can be useful in radiotherapy treatment planning for cancer patients, but can also be useful in the determination of the individual radiosensitivity for the purpose of developing educated and individualized radiation protection for healthy individuals. It may allow for the assessment of whether an individual or a tissue of an individual can be exposed to a specific radiation dose, in order to obtain, on the one hand, protection of the individual or tissue and, on the other hand, eradication of diseased tissue. In the sense of the present invention, "treatment" means that at least 90 %, preferably at least 99 %, more preferably at least 99.9 %, still more preferably at least 99.99 %, still more preferably at least 99.999% and most preferably 100 % of the cells of a diseased area, preferably a tumor, are killed.

The term "radiosensitivity" or "radiosensitive", as used herein, is the relative susceptibility of a cell, a tissue, an organ or an organism to the harmful effect of ionizing radiation, which is predominantly reflected by the radiosensitivity to killing of the comprising individual cells. The radiosensitivity of a cell is reflected inter alia by its ability to reproduce itself several times. According to the present invention, a cell, tissue, organ or organism is termed to be more radiosensitive or to have higher radiosensitivity compared to another cell, tissue, organ or organism if the number of prDSBs is higher compared to the number of prDSBs of the other cell, tissue, organ or organism. Or otherwise, according to the present invention, a cell, tissue, organ or organism is termed to be more radiosensitive or to have higher radiosensitivity compared to another cell, tissue, organ or organism if the number of tIDSBs is lower compared to the number of tIDSBs of the other cell, tissue, organ or organism. The number of prDSBs or tIDSBs is determined with respect to induction by one unit (i.e. one Gray, abbreviated as Gy) of ionizing irradiation dose indicated as prDSBs/Gray.

The term "radioresistance" or "radioresistant", as used herein, is the property of a cell or an organism to resist to ionizing radiation, without that the cell, tissue, organ or organism is killed or functionally compromised by the ionizing radiation. According to the present invention, a cell, tissue, organ or an organism is termed to be more radioresistant or to have higher radioresistance compared to another cell, tissue, organ or organism if the number of prDSBs is lower compared to the number of prDSBs of the other cell, tissue, organ or organism, or if the number of tIDSBs is higher compared to the number of tIDSBs of the other cell, tissue, organ or organism. The number of prDSBs or tIDSBs is determined with respect to induction by one unit of ionizing irradiation dose indicated as prDSBs/Gray.

The cell to be used in the method of the present invention may be any cell for which it is desirable to determine the degree of radiosensitivity. The cell may be a cell isolated from an individual, e.g. in a biopsy sample, or may be a cell present within a cell culture. In a preferred embodiment, the cell for use in the method of the present invention is isolated from an individual and is used directly, without extended storage for more than one to six hours.

The individual may be a human or an animal, preferably a human. Preferred animals are animal mammals such as dogs, cats, cattle, horses, goats, sheep, camels, rabbits, or hares. Other animals may be birds and reptiles such as chicken, goose, duck, birds of prey such as hawks, pet birds such as canary birds, parrots or budgies, crocodiles etc.

The cell to be used in the method of the present invention may be a diseased cell or the cell to be used in the method of the present invention may be a normal cell. In an embodiment of the invention, the cell is a diseased cell. The term "diseased cell", as used herein, refers to a cell which negatively influences a body and is, therefore, not wanted. The eradication of such a cell is desired, as its killing may be live-saving, or enhances the health of an organism. In a preferred embodiment, the diseased cell is characterized by an abnormal growth. In a still more preferred embodiment, the cell is a tumor cell, preferably of epithelial origin, of mesenchymal origin, of hematopoietic origin, or of neuro-ectodermal origin. Yet more preferred, the cell is selected from a breast adenocarcinoma, sweat gland adenocarcinoma, salivary gland adenocarcinoma, skin squamous cell carcinoma, adenocarcinoma of the thyroid, lung, stomach, liver, pancreas, small intestine, colon, or prostate, transitional cell carcinoma of the bladder; adenocarcinoma of the kidney, testis or endometrium, fibrosarcoma, liposarcoma, osteosarcoma, chondrosarcoma, leiomyosarcoma, hemangiosarcoma, lymphoma, leukemia, astrocytoma, retinoblastoma, oligodendroglioma, schwannoma, melanoma, head and neck cancer, ovarian cancer, adenoid carcinoma, basal cell carcinoma, epidermoid carcinoma, meningioma, neurofibroma, glioblastoma, ependymoma, medulloblastoma, neuroblastoma, hepatoma, mesothelioma, brain cancer such as glioblastoma multiforme, hepatoma, lymphoma, myeloma, neuroblastoma, sarcoma, stomach cancer, thyroid cancer, non-melanoma skin cancer, non-small cell lung cancer, cervical cancer, or anal cancer. The cell may be derived from any tumor which is known to be treatable by radiotherapy or which can be treated by radiotherapy.

Alternatively, the diseased cell may be derived from a diseased tissue, which is not characterized by an abnormal growth, however, which is amenable to radiotherapy, or for which radiosensitivity information is desired for other purposes, such as radiation protection. Examples are a diseased cell from the thyroid gland in case of the disease of Basedow or hyperthyroidism, from the pituitary gland in case of pituitary adenoma, from the meninges in case of a meningioma, from the skin with a non-cancerous skin disorder, particularly rosacea, poikiloderma of Civatte, angioma, telangiectasias, or psoriasis, or from the ankle in case of talalgia.

In a further embodiment, the cell is a normal cell. A "normal" cell is a cell without an abnormality and which is not involved in a disease. The term "normal cell" may refer to any cell of a normal or healthy organism. The term "normal cell" may refer to any cell of a diseased organism whereby the cell is not in a diseased state or is not known to be in a diseased state or whereby the cell is not involved in the disease of the organism known. The normal cell may be an epithelial cell such as a keratinocyte, or a lens epithelial cell, a melanocyte, a cardiac myocyte, a chrondrocyte, an endothelial cell, a fibroblast, an osteoblast, a preadipocyte, a skeletal muscle cell, a smooth muscle cell, a lymphocyte etc.

The determination of the degree of radiosensitivity of a normal cell may serve to distinguish between the radiosensitivities of different individuals. Moreover, the knowledge of radiosensitivity of a normal cell may help to decide to what extent an individual can be exposed to radiation. For example, based on this knowledge a decision can be reached whether an individual can work in clinical, scientific or other settings or atomic power plants with radiation load, or the decision can be made in favor of an individual with a higher radioresistance. Also, a decision can be reached whether an individual should be removed before others from contaminated areas, as this individual has a lower radioresistance and thus a higher risk for adverse effects.

Alternatively, the normal cell is derived from a diseased individual having a disease as referred to above, whereby the normal cell may be adjacent to a diseased cell of the diseased organism. The normal cell may be derived from the same tissue as the diseased cell. The determination of the degree of radiosensitivity of a normal cell and of a diseased cell may serve to enable the practitioner to develop therapeutic treatment schemes for a diseased cell, without excessively damaging a neighbored normal cell. A normal cell can be more radiosensitive than a neighbored diseased cell or can have the same radiosensitivity. In such case, if radiotherapy is used as the treatment form to treat the diseased cell, then the practitioner should take utmost care to focus radiation on the diseased cell and to spare neighbored normal cells to a maximum extent. On the other hand, a normal cell can be more radioresistant than a diseased cell. In such case, the risk to damage healthy tissue which is in the neighborhood of a diseased cell is lower and it is easier to eradicate the diseased cell while sparing the normal cell.

The term "a cell" or "the cell", as used herein, is not restricted to refer to a certain number of cells. "A/the cell" may include only "one" cell, but may also include two, three, ten, 20, 50, 100 or more cells such as several hundreds or thousands or millions of cells. The term "a/the cell" does also not pose a limitation on the isolation grade of the cell, in case the cell is isolated from an individual. Thus, the cell may be an isolated cell or isolated cells, with no other cells adhering thereto, or the cell may be present within a cell accumulation. A cell accumulation means a mass of cells, a tissue or a part thereof or an organ or part thereof isolated from an organism, wherein the cells adhere to each other. The term "a/the cell" also includes part or the whole of cells, which are present within a cell accumulation. Consequently, the term "a/the cell", as used herein, refers to a cell or to cells separated from other cells or to a cell accumulation.

A cell is isolated from an organism using methods known in the art. In a preferred embodiment, the isolated cell is processed further, i.e. is reduced in size in case of a cell accumulation, irradiated and broken down, immediately after isolation. Less preferred, the cell may be frozen or otherwise stored for further processing or may be transferred into secondary cell cultures or cell lines etc. The skilled person knows how to perform freezing, storing or transfer into secondary cell cultures or cell lines.

The present invention relates to a method for predicting the degree of radiosensitivity. The degree of radiosensitivity of a cell may be determined with respect to a reference. According to an aspect of the present invention, the term "reference" refers to a diseased individual, a diseased tissue or a diseased cell. Preferably, the reference is a diseased individual, a diseased tissue or a diseased cell if the cell to be examined is a diseased cell. Preferably, the reference individual, tissue or cell has the same disease as the diseased cell to be examined, but the reference is a different individual or is obtained from a different individual. Preferably, the reference individual, tissue or cell is known to be treatable by radiotherapy. The degree of radiosensitivity of a diseased cell to be examined may be also determined with respect to one reference diseased individual or tissue or cell derived from one diseased individual or with respect to the average of radiosensitivities of a plurality of different reference diseased individuals or reference diseased tissues or cells derived from a plurality of different diseased individuals, such as 2 to 200, 3 to 100, 3 to 50 or 5 to 10 different diseased individuals. The comparison of the radiosensitivity of a diseased cell to be examined with a diseased individual, tissue or cell may help to decide whether it is sensible to irradiate a diseased tissue or to choose another treatment form. If the radiation dose for treating a diseased tissue resulting in treatment of the disease is known, the comparison may allow to choose the correct radiation dose for treatment of the disease. Most preferably, the reference is a cell from the same type as the cell to be examined, however, from (a) different individual(s).

In a preferred embodiment, the cell to be examined is a tumor cell and the reference is a reference tumor or reference tumor cell. In an embodiment thereof, the reference tumor or reference tumor cell is from the same tumor type as the tumor cell to be examined, albeit from a different individual, or the reference tumor or reference tumor cell is from a different tumor type or a plurality of different tumor types. Preferably, the reference tumor or reference tumor cell is known to be treatable by radiotherapy. The number of prDSBs of the reference tumor or reference tumor cell may be known or may be determined along with the determination of the radiosensitivity of the tumor cell to be examined or treated, according to the method of the present invention. The degree of radiosensitivity may be determined with respect to one reference tumor or to a reference tumor cell derived from one tumor or with respect to the average of radiosensitivities of a plurality of different reference tumors or of reference tumor cells derived from a plurality of different tumors, such as 2 to 200, 3 to 100, 3 to 50 or 5 to 10 different tumors. For example, a reference may be the average radiosensitivity of a plurality of different tumor types or tumor cells derived from different tumor types and this average radiosensitivity may serve as a classifying parameter to classify a tumor or tumor cell as radiosensitive or radioresistant. Thus, if the number of prDSBs is higher in a tumor cell as compared to the average number of prDSBs from the reference, then the tumor cell may be classified as radiosensitive and this may motivate the practitioner to treat the tumor by radiotherapy using lower total radiation doses, thus reducing damage to healthy tissues. Preferably, the average radiosensitivity of the reference tumors reflects the average radiosensitivity of tumor cells in general. Comparison of radiosensitivities of tumor cells from different tumor types requires that same or principally same conditions are used for the determination of the number of prDSBs. This means that the cells have to be treated before or during irradiation and during the further processing steps (disruption of cells and determination of the number of prDSBs) in the same manner. Differences in experimental conditions should account to special requirements of a cell such as different isolation or disruption methods etc. The above is also applicable to diseased cells other than tumor cells which are treatable by radiation.

According to another aspect of the present invention, the term "reference" refers to a normal cell, a normal tissue or a normal individual. The term "normal tissue" or "normal individual" may refer to any tissue or individual that is not in a diseased state, or that is not known to be in a diseased state. Preferably, the reference is a normal individual, a normal tissue or a normal cell if the cell to be examined is also a normal cell. Preferably, the normal cell is an epithelial cell such as a keratinocyte or a lens epithelial cell, a melanocyte, a cardiac myocyte, a chrondrocyte, an endothelial cell, a fibroblast, an osteoblast, a preadipocyte, a skeletal muscle cell, a smooth muscle cell, a lymphocyte etc. More preferably, the cell to be examined and the reference cell are of the same type, e.g. both cells are epithelial cells such as keratinocytes. Moreover, the individual from which the cell to be examined is obtained may be different from the individual from which the reference cell is obtained. Thus, in an especially preferred embodiment, the cell to be examined and the reference cell are of the same type, however, from different individuals. A comparison of the degree of radiosensitivity of a normal cell or normal individual with respect to a normal cell from another individual or to another normal individual as reference may help to decide which individuals to select, e.g., for an activity associated with an exposition to radiation. Preferably, one would select an individual with higher radioresistance for an employment in an area with radiation load such as in clinical or scientific settings or in an atomic power plant. On the other hand, in case of an atomic accident, individuals with a lower degree of radioresistance as compared to a reference are preferably removed before others from the contaminated area. The reference may be a normal cell derived from one individual, so that the comparison is between an individual and the reference individual, or the reference may be a plurality of tissues or cells derived from different individuals, preferably derived from the average population, such as 2 to 200, 3 to 100, 3 to 50 or 5 to 10 individuals, to obtain an average radiosensitivity as reference value. Preferably, the tissues or cells to be compared are of the same type.

The comparison of the radiosensitivity of a cell, tissue or individual to a reference also includes the comparison of a diseased cell, e.g. having a disease as referred to above, with a normal cell as reference cell. The comparison may help to decide which radiation dose to apply to treat a disease. Preferably, both the diseased cell and the normal cell are from the same individual and/or are from the same tissue. More preferably, the diseased cell is adjacent to the normal tissue and is derived from the same tissue. If the radiosensitivity of the diseased cell, e.g. tumor cell, is higher than that of the adjacent normal cell, then the diseased cell may be irradiated with a radiation dose which allows the treatment of the disease, however, which is low enough not to damage, e.g. kill, or functionally impair the adjacent normal tissue, or to damage the adjacent normal tissue only to an extent which is far below the damage of the diseased tissue, such as below 30 %, 20 %, 15 %, 10 %, 5 % or is even 0 %. If the radiosensitivity of the diseased cell is lower than that of the normal cell, then application of such high radiation doses may be necessary which also damage the adjacent healthy tissue. In such case, a practitioner may decide to treat the diseased cell by other treatment forms or the practitioner must use utmost care in order not to damage or to damage only to a minor extent, as indicated above, adjacent normal tissue by exposing a patient to radiation from different directions using different fields.

The term "degree of radiosensitivity" is used herein in the context of relationship of radiosensitivities of different cells. A cell may be more radiosensitive or less radioresistant or it may be less radiosensitive or more radioresistant than a different cell. For example, as can be taken from Figure 5, the cell line HT144 is more radiosensitive than the cell line SQ20B, which is more radioresistant.

Irradiation of a cell is performed in the method of the present invention with any type of radiation, preferably with a type of radiation which is used in medical practice to treat diseases such as tumor or cancer. Preferably, the type of radiation is ionizing radiation. Ionizing radiation represents a type of radiation that carries energies high enough to cause ionizations leading to chemical alterations. IR includes photon radiation such as X radiation, gamma radiation, cathode radiation, or radiation with particles such as alpha or beta particles, neutrons, protons or heavy ions. Preferably, the kind of IR used in the method of the present invention is X radiation.

The unit of the IR dose adsorbed by a mass of material is the unit Gray (Gy) which is defined as the energy deposited in a defined mass of material. Thereby, one Gy is equal to 1 Joule/kg.

According to the present invention, radiosensitivity of a cell is determined by the number of prDSBs which are induced by the application of ionizing radiation. Thereby, the amount of the irradiation dose (in Gray) resulting in the same number of prDSBs may be different between different cell types. According to the present invention, irradiation of a cell in order to determine the degree of radiosensitivity thereof is carried out at an irradiation dose which results in damage, preferably killing, of the cell. In a preferred embodiment, the irradiation dose used in the method of the present invention is larger than 0 to 50 Gray, preferably larger than 0 to 30 Gray, more preferably larger than 0 to 20 Gray, and still more preferably larger than 0 to 15 Gray. "Larger than 0" includes any dose above 0 which results in a damage such as killing of a cell, such as 0.1 or preferably 0.5 Gray.

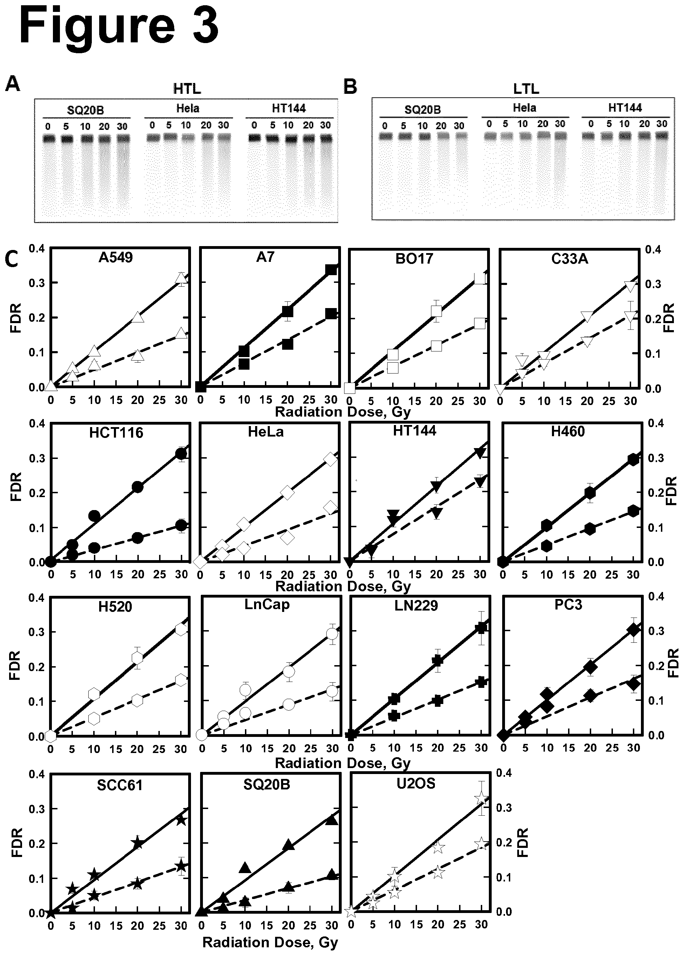

In an embodiment of the present invention, a cell is irradiated with one selected irradiation dose for determining the number of prDSBs. As can be taken from Figure 5 A and B and Figure 6, the number of prDSBs for a given cell remains constant for the ratio prDSBs per Gray (prDSBs/Gray). In another embodiment, a cell may be irradiated in parallel experiments, such as 2, 3, 4, 5, or 6 or more experiments, with a different irradiation dose per experiment. The application of different irradiation doses in parallel experiments allows the generation of linear dose response-curves (see e.g. Figures 3 and 4) which allow a precise regression analysis for the determination of prDSBs.

Irradiation should be carried out under conditions allowing the formation of prDSBs, however, preventing the formation of tIDSBs; i.e. under conditions at which thermally labile sugar lesions are not generated and, in case of generation, at which thermally labile sugar lesions do not convert to breaks so that tIDSBs are not produced. Moreover, the conditions should be selected such that there is no onset of DNA repair mechanisms of the damaged DNA. This may be achieved by selecting a suitable temperature and a suitable time range of irradiation. The skilled person will be aware that the higher the temperature and the longer the time of irradiation, the higher the probability that tIDSBs and/or DNA repair mechanisms are induced.

Therefore, in an embodiment of the present invention, irradiation is carried out at any temperatures which prevent the formation of tIDSBs and the onset of DNA repair mechanisms of the damaged DNA, preferably at 0 to 20°C, more preferably at 0 to 10°C, still more preferably at 0 to 6°C and most preferably irradiation is carried out on ice or at 0°C. When irradiation temperatures lying above 20°C are used, then formation of tIDSBs and onset of DNA repair mechanisms may be induced, resulting in the determination of total DSBs (tDSBs), being the sum of prDSBs and tIDSBs. Moreover, prDSBs may be repaired at higher temperatures such as temperatures above 20°C, so that not the actual number of prDSBs resulting from irradiation is measured and the result will be falsified.

In a further embodiment, irradiation is carried out within a continuous time range without interruptions or is carried out with interruptions. Preferably, irradiation is continuous. Continuous irradiation may be carried out within 1 min to 24 h, preferably 1 min to 12 h, more preferably 1 min to 2 h or even more preferably 1 min to 1 h, or most preferably within 1 to 5 min. Less preferred, irradiation may be carried out in a discontinuous manner within 10 min to 6 hours, preferably 5 min to 2 hours, allowing intervals of 5 min to 4 hours, but in such cases cells must be maintained on ice.

In a preferred embodiment, irradiation is carried out at a total irradiation dose of 0.5 to 50 Gray within a time range of 5 min to 1 hour and at a temperature of 0 to 20°C, more preferably irradiation is carried out at an irradiation dose of 0.5 to 30 Gray within a time range of 5 min to 45 minutes and at a temperature of 0 to 10°C, still more preferably irradiation is carried out at an irradiation dose of 0.5 to 20 Gray over a time range of 30 min and at a temperature of 0 to 6°C.

The cell to be irradiated may be present as a separated single cell such as a single cell suspension comprising one or more than one cell or may be present as a cell accumulation. Preferably, the cell to be irradiated is present as a single cell such as a single cell suspension. If the cell is present in a single cell suspension state, the cell density of the suspension is such that essentially (i.e. at least 95, more preferably 97, more preferably 98, more preferably 99 or most preferably 100 %) all cells comprised by the suspension are irradiated, preferably the cell density amounts to 0.01 million to 10 million cells per milliliter or more preferably 0.1 million to 10 million cells per milliliter, or even more preferably 1 million to 10 million cells per milliliter. If the cell is present as a cell accumulation, such cell accumulation preferably comprises such a number of cells that uniform irradiation of essentially each cell comprised by the cell accumulation is ensured, preferably being not more than 10 million cells, more preferably not more than 5 million cells, still more preferably not more than 2 million cells, and still more preferably not more than 1 million cells. If the cell has been isolated as an accumulation of cells, it may be required that this accumulation of cells has to be reduced to smaller pieces. Alternatively, the cell accumulation may be cautiously flattened, so that the cells are present in thin layers. The objective of the reduction or flattening of a cell accumulation is to allow a uniform irradiation of essentially (i.e. at least 95, more preferably 97, more preferably 98, more preferably 99 or most preferably 100 %) all cells comprised by the cell accumulation, which is more difficult if the cells cover each other as thick layers. Methods for reducing the size of cell accumulations and/or to prepare single cell suspensions are known in the art. The method depends on the kind of tissue. The skilled person knows what method to use in order to reduce the size of a cell accumulation. Examples are mechanical methods such as mincing or shearing of the cell accumulation by using a scalpel, knife or scissors or disaggregating the cell accumulation with tweezers, pressing the cells from each other, pushing through a screen, or trituration by pipette. If an accumulation of cells is used in the method of the present invention, this accumulation may be pressed apart to ensure that the cells form a flat layer, so that the cells are uniformly irradiated. An additional or alternative method is enzymatic disaggregation using enzymes such as trypsin, collagenase, protease, hyaluronidase, heparinase and/or dispase. Disaggregating a cell accumulation may be done in a suitable buffer, as is known in the art. Examples are tissue culture media, balanced salt solutions, growth media, serum-free growth media etc. Temperatures for performing mechanical methods may be from 0 to 30°C, preferably from 0 to 20°C, more preferably from 0 to 10°C, and still more preferably on ice. Temperatures for enzymatic disaggregation depend on the kind of enzyme and may be 10 to 50°C, preferably 20 to 40°C, more preferably 30 to 40°C, and still more preferably 37°C.

After the isolated cell has been brought into a state allowing a uniform irradiation, irradiation is performed. In a preferred embodiment, the single cell such as a single cell suspension or the cell accumulation is poured as a plug before irradiation, preferably with a diameter of 1 to 10 mm, more preferably of 2 to 6 mm and still more preferably of 3 mm and preferably with a height of 1 to 15 mm, more preferably of 2 to 10 mm and still more preferably of 5 mm. Particularly preferred are plugs with a diameter of 3 mm and a height of 5 mm. The plug may of course also take other forms such as a cuboid with sizes corresponding to those indicated above. The polymerizing substance for hardening the suspension or enclosing the cell accumulation may be any polymerizing substance which is suitable for encapsulating cells, such as agarose, preferably low-melting agarose. The end concentration of the polymerizing substance within the plug is 0.1 to 2% by weight. The cell density or the number of cells of a cell accumulation is indicated above. Irradiation may also be performed on a cell or cell accumulation which is present in suspension or which is present on a medium such as a plate, a petri dish, a slide, a polymerized medium. If the cell is present as a single cell suspension, the amount of suspension which is irradiated is 0.1 to 10 milliliter, preferably 0.1 to 5 milliliter or even more preferably 0.1 to 2 milliliter.

In a preferred embodiment of the present invention, irradiation is performed in vitro, after the cell has been isolated from an organism. This ensures that the number of prDSBs which form as a direct result of irradiation can immediately be determined, without that the cell needs to be isolated from the body. Isolation after irradiation may bear the risk of onset of DNA repair mechanisms or induction of tIDSBs, and this is reduced or totally eliminated when the cell is, immediately after irradiation, processed further, i.e. the cell is disrupted and prDSBs are determined as described above.

After irradiation, the measurement of prDSBs is performed. Preferably, measurement is immediately performed after irradiation. This means that any steps which are necessary for measurement such as disrupting the cell and determination of DSBs are immediately performed after irradiation, without that the cell is stored in between. If it is not possible to process the cell immediately after irradiation and the cell must be stored, then storage is to be performed under conditions which prevent the formation of tIDSBs and the onset of DNA repair mechanisms in the damaged DNA. Such conditions preferably include low temperatures such as 0 to 20°C, more preferably 0 to 10°C, most preferably 0°C. Alternatively, the cell may be frozen. However, the freezing or cooling process must not essentially change the number of DSBs, whereby "essentially" means that the number of DSBs which are present immediately after irradiation is not changed or is changed by 10 %, 5 %, 3 % or 2 % at the most.

After irradiation, the cell is disrupted in order to liberate the DNA for determining the number of prDSBs. For disrupting the cells, in principle any cell disruption technique may be used, as long as the DNA is preferably not damaged, or the DNA is only damaged to a low amount such as 5 %, preferably 1 %, more preferably 0.5 %, still more preferably 0.3 % or still more preferably 0.2 % at the most by this procedure. Examples of cell disruption techniques are different forms of lysis. Preferably, the cells are subjected to a lysis process in order to cautiously liberate the high molecular weight DNA from the cells. Lysis of a cell can be performed in any lysis buffer using any kind of lysis mechanisms. Examples of different lysis mechanisms are the use of detergents, or viral, enzymatic, or osmotic mechanisms. Preferably, the mechanism underlying the lysis process used in the present invention is the use of detergents. Lysis solutions are known in the art. A typical lysis buffer contains salts (e.g. Tris-HCI, EDTA) to regulate the acidity and osmolarity of the lysate, while detergents (e.g. N-Lauryl (N-lauryl-sarcosine (NLS)), Triton X-100, SDS) are added to break up membrane structures. A typical lysis buffer for use in the present invention comprises EDTA and NLS. Preferably, disruption such as lysis is performed if the cell is present within the plug, as the presence in a plug avoids shearing and breaking of the high molecular weight DNA liberated from the cell. Disruption is performed at temperatures which prevent the formation of tIDSBs and the onset of DNA repair mechanisms of the damaged DNA, preferably in the range of 0 to 20°C, more preferably in the range of 0 to 10°C, still more preferably in the range of 2 to 6°C and most preferably at 4°C. If disruption temperatures lying above such temperatures, such as in the range of above 20 to 70°C, 30 to 60°C, 40 to 55°C, or 50°C are applied, then formation of tIDSBs and onset of DNA repair mechanisms may be induced, resulting in the determination of total DSBs (tDSBs), being the sum of prDSBs and tIDSBs. The time range for disrupting the cell is such that essentially all cells which are present in the disruption medium are disrupted, for example 10 to 30 h, depending on the disruption process used. The term "essentially" in this context means that at least 90 %, preferably at least 95 %, more preferably at least 97 %, still more preferably at least 98 %, still more preferably at least 99 % and most preferably 100 % of the cells are disrupted.

After disrupting the cell, the liberated DNA is used for the determination of prDSBs. Preferably, the whole plug comprising the broken cell is used. If the cell is not embedded in a polymerized medium during irradiation, the liquid medium comprising the disrupted cell including the liberated DNA may be used. Alternatively, the DNA may be purified from the liquid medium comprising the disrupted cell using any method known in the art. Thereby, the cell is treated after irradiation under conditions that the integrity of the liberated DNA is maintained and the DNA is preferably not damaged or the DNA is damaged to only a low extent exceeding not more than 5 %, preferably 1 %, more preferably 0.5 %, still more preferably 0.3 % or still more preferably 0.2 %.

For determining the number of prDSBs, any method known in the art may be used. Preferably the method used is electrophoresis, more preferably pulsed-field gel electrophoresis, and still more preferably asymmetric field inversion gel electrophoresis. Pulsed-field gel electrophoresis (PFGE) is one of the most reliable methods for detecting DNA double-strand breaks. If the cell is lysed within a plug, the plug is inserted into the well of the electrophoresis gel. If the cell has been broken up in the liquid medium, the medium including the liberated DNA is inserted into the well of the electrophoresis gel. If the DNA has been purified from the liquid medium and resolved in a new medium, then this medium is inserted into the well of the electrophoresis gel. The number of prDSBs may be indirectly measured and indicated by the fraction of DNA released (FDR) out of the well into the lane (28-32). The released DNA corresponds to fragmented DNA, which is the result of prDSBs. FDR is indicated in percent. The method of measuring FDR is well-known in the art (37, 38). Based on FDR, the actual number of prDSBs can be determined using theoretical calculations (31) or calibration using disintegration of ¹²⁵I incorporated into DNA, for which is known that one disintegration causes the induction of one DSB (37). For the assessment of the degree of radiosensitivity of a cell, the determination of the FDR may be sufficient. The FDR values may be compared to a reference and the degree of radiosensitivity as compared to the reference can thus be determined. As an example, the FDR values obtained for tumor cell lines as depicted in Figure 4 may be compared to each other and the relationships of the radiosensitivities may be indicated. A further example of a method for determining prDSBs is the Comet assay. The Comet assay is a single cell gel electrophoresis assay for the detection of DNA damage. A further method is constant-field gel electrophoresis (CFGE), which can also detect DSBs. All the electrophoresis methods mentioned above are well-known in the art and the skilled person knows to apply these methods to determine the number of prDSBs in the DNA of a cell.

In a further aspect of the present invention, the present invention comprises the determination of the number of tIDSBs for determining the degree of radiosensitivity of a cell. The present inventors have shown that the number of prDSBs of different tumor cell lines correlates with the surviving fraction of the cells in the colony formation assay and is, thus, a measure of the degree of the radiosensitivity of a cell. Due to the presence of similar amounts of tDSBs in the tumor cell lines treated under conditions to allow the formation of tIDSBs (see Figure 4 A), one may conclude that the number of tIDSBs, which can be calculated by subtracting the number of prDSBs from tDSBs, is also predictive of the degree of radiosensitivity of a cell. Thus, while a higher number of prDSBs identifies a cell as being more radiosensitive as compared to a reference, a higher number of tIDSBs identifies this cell as being more radioresistant as compared to a reference.

Based on the above, the present invention relates to an in vitro method for predicting the degree of radiosensitivity of a tumor cell, comprising irradiating a tumor cell, determining the number of thermally labile sugar lesion-dependent double-stand breaks (tIDSBs) in the tumor cell, and using the number of tIDSBs to predict the degree of radiosensitivity of said tumor cell.

For determining the number of tIDSBs, the skilled person determines the number of tDSBs and subtracts therefrom the number of prDSBs. Determination of the number of prDSBs is described above. The number of tDSBs may be determined by any method known in the art. Preferably, the number of tDSBs is determined, in principle, in the same manner as described above for the determination of the number of prDSBs. However, disruption of the cell is performed at temperatures allowing the formation of tIDSBs, such as temperatures above 20°C, for example 20 to 70°C, 30 to 60°C, 40 to 55°C, or 50°C. The time range for disrupting the cell is such that it correlates with the disruption process - the disruption process is performed at a temperature above 20°C to allow formation of tIDSBs - and that essentially all cells which are present in the disruption medium are disrupted, for example 10 to 30 h, depending on the disruption process used. The term "essentially" in this context means that at least 90 %, preferably at least 95 %, more preferably at least 97 %, still more preferably at least 98 %, still more preferably at least 99 % and most preferably 100 % of the cells are disrupted.

The methods of the present invention may be used for determining the radiation dose for treating a tumor in an individual. In order to achieve this, the number of prDSBs or tIDSBs of a tumor cell or tumor (e.g. indicated as prDSBs/Gray or tIDSBs/Gray) to be examined or to be treated may for example be compared with number of prDSBs or tIDSBs of a reference tumor (e.g. indicated as prDSBs/Gray or tIDSBs/Gray) which reference tumor is known to be treatable by radiotherapy and for which reference tumor the radiation dose for treating the reference tumor is known. Thus, if the number of prDSBs or tIDSBs of the tumor to be examined or to be treated is similar to the number of prDSBs or tIDSBs of the reference tumor, then the practitioner may assume that same or similar irradiation doses might be efficient to treat the tumor. However, if the number of prDSBs or tIDSBs largely differs from the number of prDSBs or tIDSBs of the reference tumor, then the practitioner may assume that the irradiation doses efficient to treat the tumor have to be much higher or may be advantageously much lower than those for the reference tumor. Moreover, if it turns out that a tumor to be examined or to be treated is more radioresistant than the reference tumor, thus requiring high irradiation doses for treatment, the practitioner may decide in favor of a different treatment method. Consequently, correlating the number of prDSBs or tIDSBs of a tumor to be examined or to be treated with the number of prDSBs or tIDSBs of a reference tumor does not only allow the practitioner to decide whether radiotherapy is the suitable treatment form for a tumor, but also to decide on the extent of the radiation dose which is necessary to treat the tumor.

### Figures

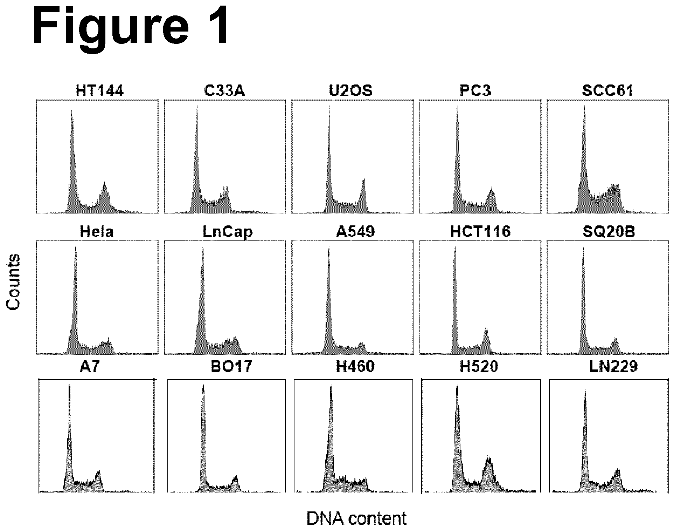

Figure 1: Representative flow cytometry histograms of the 15 cell lines used to generate the results described in the present invention, fitted with WinCycle to evaluate the distribution of the cells throughout the cell cycle. The calculated percentage of cells in G1, S and G2 phases of the cell cycle is summarized in Table 1.

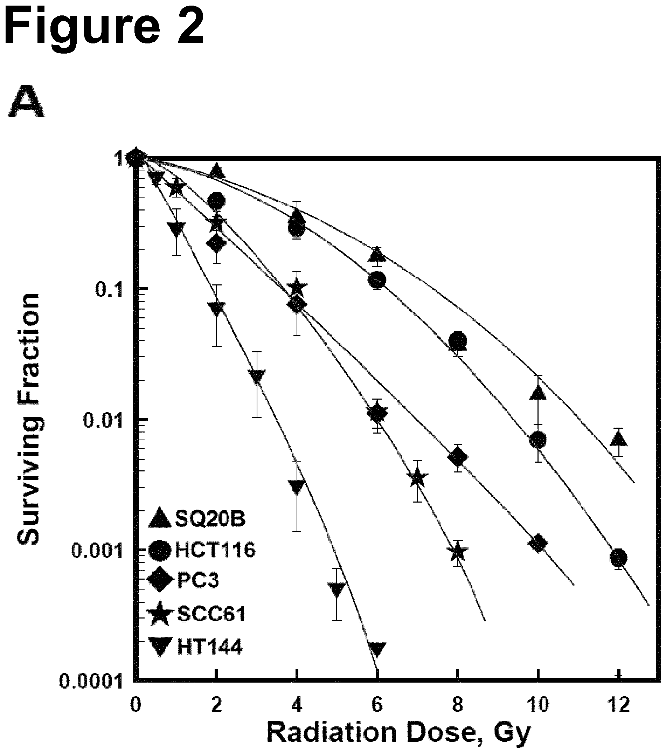

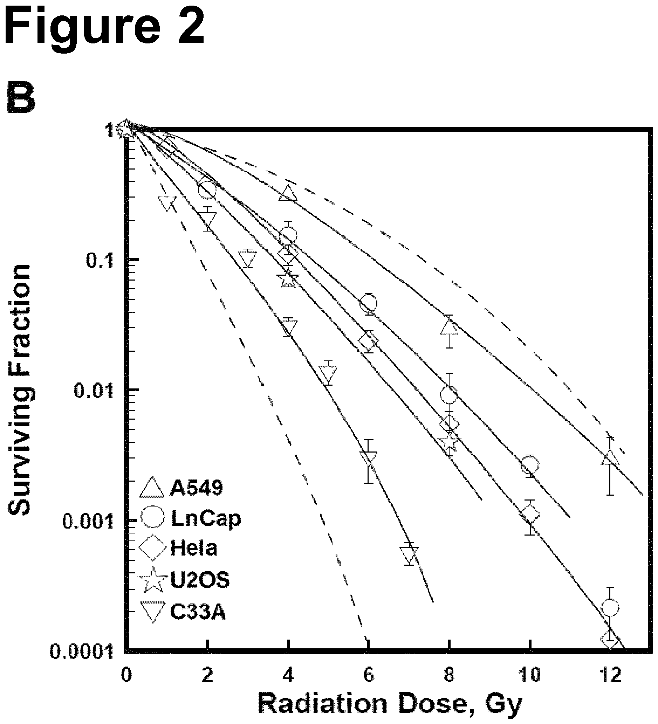

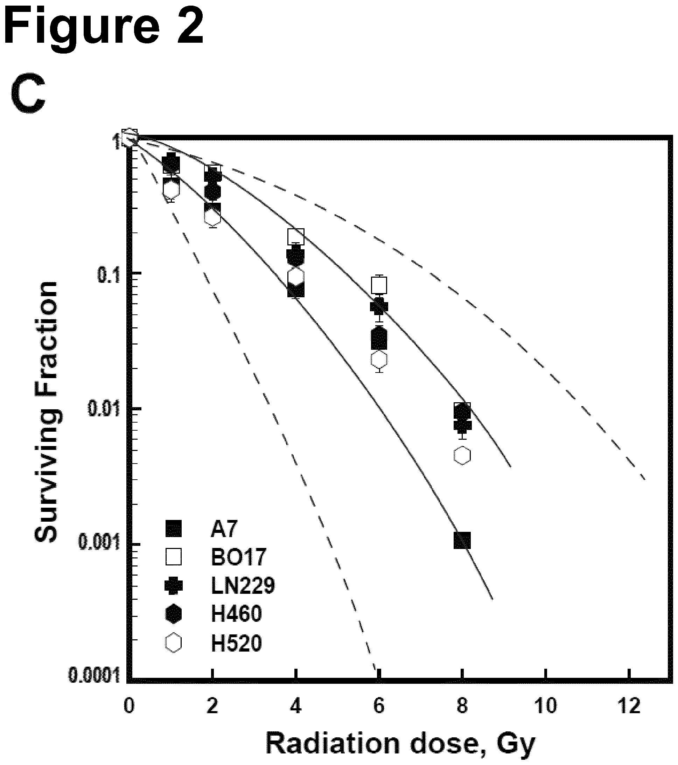

Figure 2: Panel A, Panel B und Panel C: Survival curves of the indicated cell lines obtained using exponentially growing cells and colony formation as endpoint. Results shown represent the mean and standard deviation calculated from 8 determinations in 2 experiments. The lines shown were fitted to the data points by eye. The different cell lines are allocated in the three panels aiming to maximize clarity. Broken lines in B and C show the response of HT144 and SQ20B cells and have been transferred from A to facilitate comparison.
Figure 3: Yields of tDSBs measured using high temperature lysis and of prDSBs measured using low temperature lysis. Cells in the exponential phase of growth were embedded in agarose blocks, irradiated and processed immediately thereafter. *Panel A:* Representative images of gels stained with ethidium bromide after high temperature lysis for the indicated cell lines. *Panel B:* As in A after low temperature lysis. *Panel C*: Dose response curves measured after high temperature lysis (solid lines) and low temperature lysis (broken lines) in the different cell lines. Results shown represent the mean and standard deviation calculated from 6 determinations in 2 experiments. The lines shown are linear regressions through the measured points of each data set. The slopes of these lines are used in the comparison of the DSB-yields under different conditions and are also summarized in Table 1.
Figure 4: *Panel A:* Compilation of all dose response curves obtained with the different cell lines using high temperature lysis. *Panel B:* Compilation of all dose response curves obtained with the different cell lines using low temperature lysis. *Panel C*: Compilation of all dose response curves obtained with the different cell lines specifically for the induction of tIDSBs. These results were obtained by subtracting from HTL-FDR values LTL-FDR values. Results have been compiled from the data shown in Figure 3. The list reflects the sequence of the lines in the figures from top to bottom.
Figure 5: Correlation between induction of prDSBs, represented here by the slope of the corresponding dose-response curve (Figures 3 and 4), as a function of the radiation dose required for a survival level of 37% (Panel A) and 10% (Panel B). Each circle represents one cell line and is labelled with its corresponding name. *Panel C*: As in panel B but for the yields of tDSBs (Figures 3 and 4).
Figure 6: Correlation between inductions of prDSBs, represented here by the slope of the corresponding dose-response curve (Figures 3 and 4), as a function of the radiation dose required for a survival level of 1%. Each circle represents one cell line and is labelled with its corresponding name.

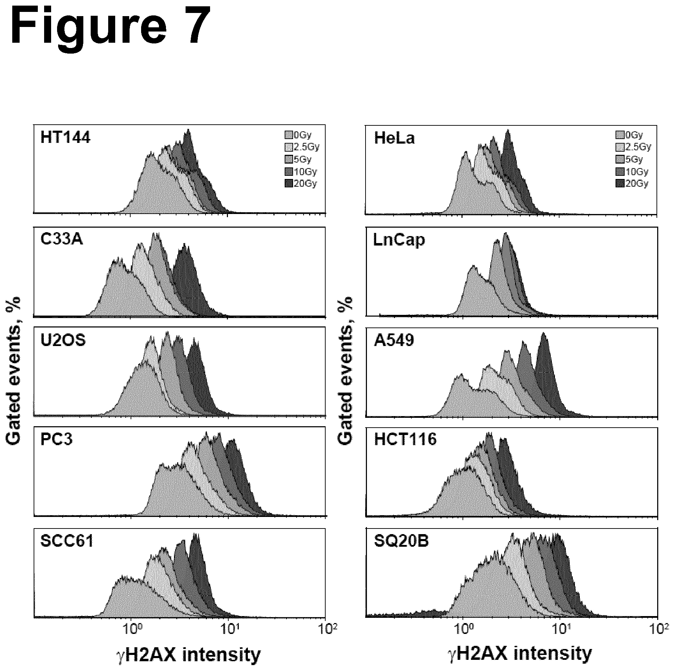

Figure 7: Representative flow cytometry histograms depicting the changes of γ-H2AX fluorescent signal as a function of radiation dose for ten of the cell lines tested using this assay.

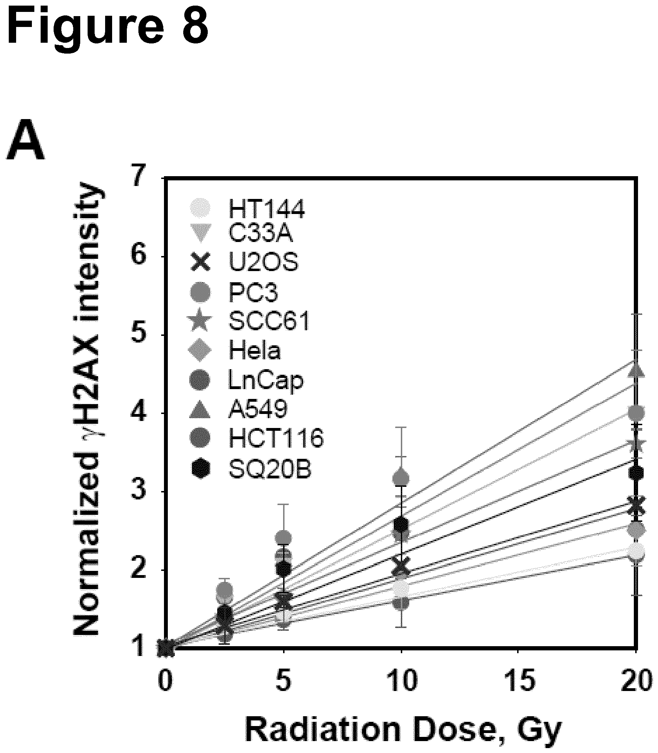

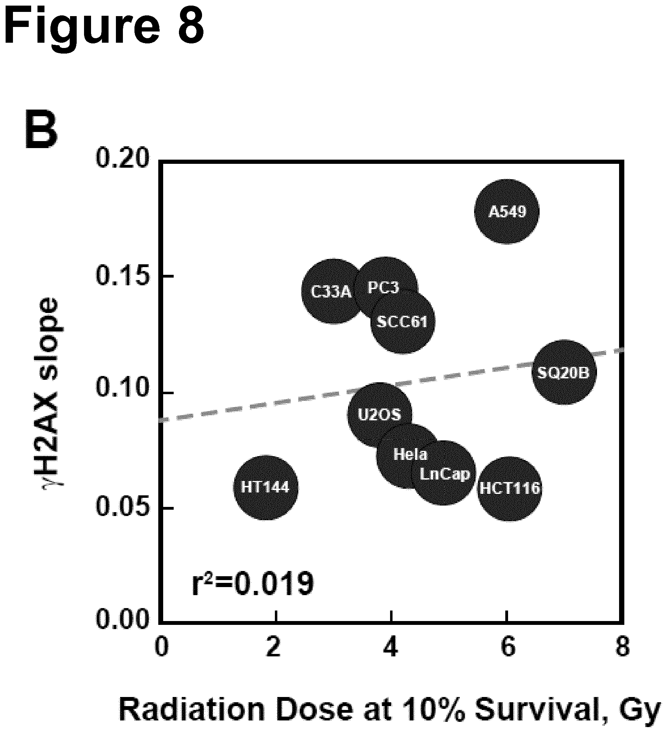

Figure 8: *Panel A:* Normalized γ-H2AX signal intensity, measured by flow cytometry 1 h after IR, plotted versus radiation dose for the indicated ten cell lines. Results from three independent experiments are shown as mean values and standard deviations. Results are also normalized for the DNA content of each cell line. *Panel B:* Correlation between yields of γ-H₂AX signal, i.e. slope of the corresponding dose-response curve in *Panel A,* as a function of radiation dose required for a survival level of 10% for each of the cell lines tested.

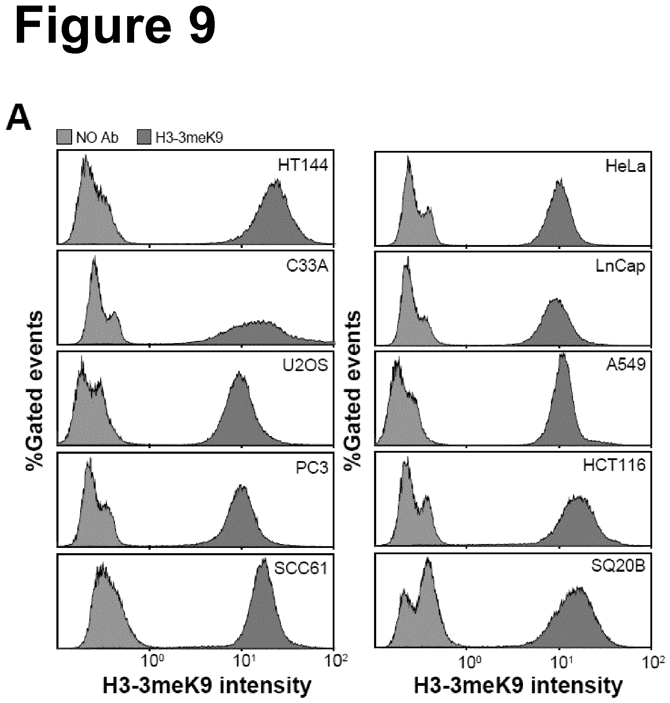

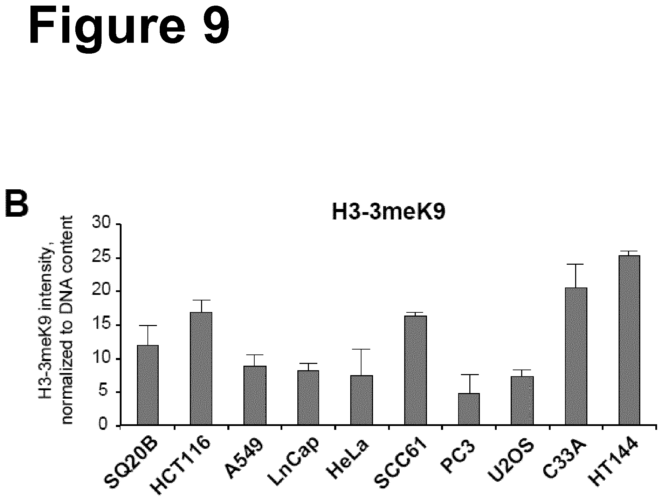

Figure 9: *Panel A:* Representative flow cytometry histograms showing the signal of Histone H3 3meK9 in ten of the cell lines tested with this assay. Shown in the left of each individual panel are the histograms of cells incubated only with secondary antibody. *Panel B:* Bar plots representing the intensity of Histone H3 3meK9 signal, normalized to DNA content. The results are means from three independent determinations and the error bars represent standard deviations.

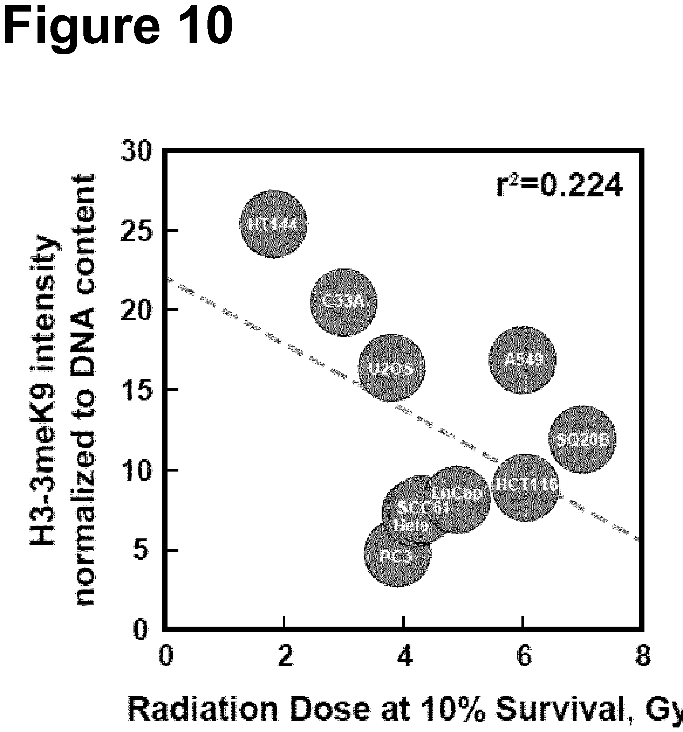

Figure 10: Normalized (to the DNA content of each cell line) fluorescent signal measured by flow cytometry of histone H3-3meK9, a measure of heterochromatin, versus radiation dose required for a survival level of 10% for ten of the cell lines tested here.

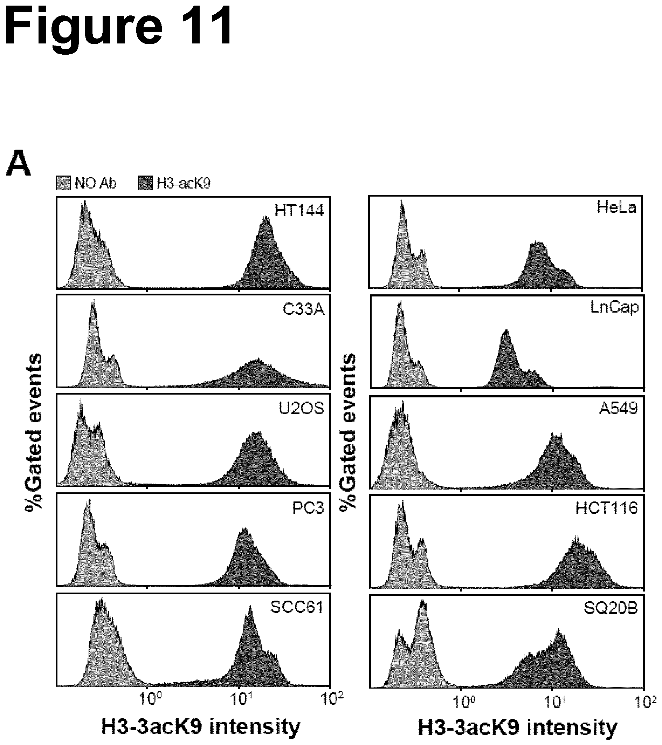

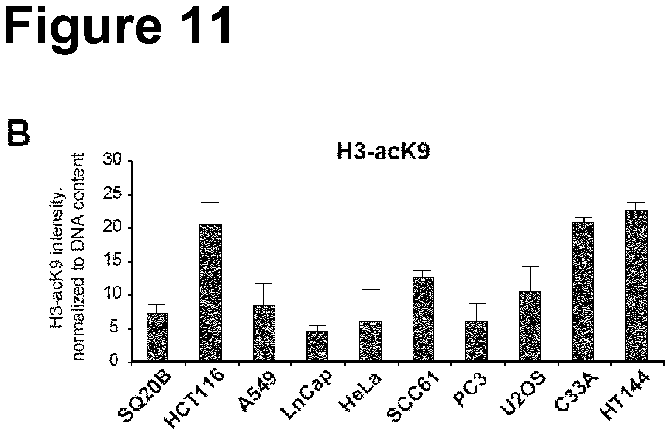

Figure 11: *Panel A and Panel B:* As in Figure 9, but for Histone H3 acK9.

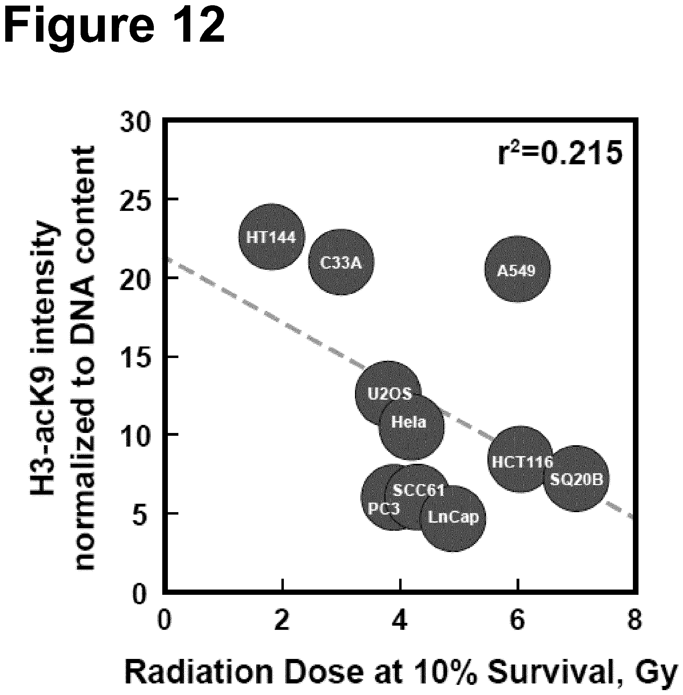

Figure 12: As in Figure 10, but for Histone H3 acK9.

### Experiments

### Materials and Methods

### Cell culture and irradiation

For experiments we employed: The human cervical epithelial carcinoma cell lines HeLa and C33A; the human melanoma cell line HT144; the human prostate epithelial cancer cell lines PC-3 and LnCap and the human colorectal carcinoma cell line HCT116. This group of cell lines was grown in Minimum Essential Medium (MEM), supplemented with 10% fetal bovine serum (FBS). In addition we employed: The human lung adrenal carcinoma cell line A549, and the human osteosarcoma cell line U2OS, which were grown in McCoy's 5A medium, supplemented with 10% FBS. We finally employed: Two human head and neck squamous carcinoma cell lines, SQ20B and SCC61, which were maintained in Dulbecco's Modified Eagle's Minimum Essential Medium (D-MEM) supplemented with 10% FBS. All cell lines were maintained at 37°C in 5% CO₂ and were used in the exponential phase of growth. Three human glioma cell lines (A7, Bogdahn 17, LN229) were grown in Minimum Essential Medium (MEM), supplemented with 15% fetal bovine serum (FBS) and 1% non-essential amino acids. The group of two human non-small cell lung carcinoma cell lines (H460, H520) was maintained in Roswell Park Memorial Institute (RPMI 1640) medium supplemented with 10% FBS.

Radiation exposures were carried out in parallel with multiple cell lines using both lysis protocols in two X-ray units (Precision X-ray, North Branford, CT) operated at 320 kV, 10 mA with a 1.65 mm Al filter. They were carried out on ice to prevent repair in experiments measuring DSB induction, and at room temperature in cell survival experiments.

### Colony formation assay

Standard procedures were used to measure colony formation. For example, after exposure to different radiation doses, cells in exponential phase were trypsinized and plated into 60 mm tissue culture dishes at increasing numbers with increasing radiation exposure aiming for 20-200 colonies per dish. They were stained with crystal violet two weeks later and counted. Clones with more than approximately 50 cells were considered to originate from surviving cells.

### Pulsed-field gel electrophoresis

Induction of DSBs was measured by Asymmetric Field Inversion Gel Electrophoresis (AFIGE), a Pulsed-Field Gel Electrophoresis (PFGE) technique as previously described (29, 31-33), using, as appropriate, for the same pool of agarose blocks either high (50°C) (HTL) or low (LTL) (4°C) temperature during lysis. In this assay, the number of DSBs present in cells is indirectly measured by the fraction of DNA released (FDR) out of the well into the lane (38).

Briefly, cells were trypsinized, suspended in serum-free HEPES-buffered growth medium and mixed with 1% low-melting agarose (Bio-Rad). The agarose cell suspension was pipetted into glass tubes of 3 mm diameter and was allowed to solidify on ice before removing from the glass tube and cutting into 5 mm blocks (plugs), which were transferred for irradiation to tissue culture dishes containing serum-free growth medium.

After irradiation, plugs were transferred to lysis solution and lysed either at high (50°C) (HTL) or low (LTL) (4°C) temperatures. For HTL, plugs were lysed in a solution containing 10 mM Tris-HCI, 100 mM EDTA, pH 7.6, 2% N-lauryl (NLS) and 0.2 mg/ml protease added just before use, at 50°C for 18 h. For LTL, plugs were first transferred into ESP buffer (0.5 M EDTA, pH 8.0, supplemented with 2% NLS and 1 mg/ml protease, both added just before use) for 24 h, and subsequently into a high-salt buffer (4 mM Tris, pH 7.5, 1.85 M NaCl, 0.15 M KCI, 5 mM MgCl₂, 2 mM EDTA and 0.5% Triton-XI00 added just before use) for 16 h.

Electrophoresis was carried out after RNAase treatment in gels, prepared with 0.5% molecular biology grade agarose (Bio-Rad), at 8°C for 40 h applying 50 V (1.25 V/cm) for 900 s in the forward direction and 200 V (5.00 V/cm) for 75 s in the reverse direction. Gels were stained with ethidium bromide and imaged in a fluor-imager (Typhoon, GE Healthcare). FDR was analyzed using ImageQuant 5.2 (GE Healthcare). Calculations of absolute numbers of DSBs (either tDSBs or prDSBs), when desired, were based on previously described calibrations (29, 37). For comparisons between cell lines, however, such absolute calculations of the numbers of induced DSBs are not required.

### DSB analysis via flow-cytometry-quantification of γ-H₂AX

Samples containing 2-3×10⁶ cells for selected cell lines were suspended for 5 min on ice in phosphate buffered saline (PBS) containing 0.2% Triton X-100. Cells were fixed for 15 min in PBS containing 3% paraformaldehyde and 2% sucrose, and were incubated in PBG blocking buffer (0.5% BSA, 0.2% gelatin in PBS) overnight at 4°C. Cells were incubated for 1 h at RT with an antibody against γ-H2AX (GeneTex) diluted in PBG, and subsequently for 1 h with a secondary antibody conjugated with AlexaFluor647. DNA was stained with propidium iodide and samples were analyzed in a flow cytometer (Galios, Beckman-Coulter, USA).

### Analysis by flow cytometry of euchromatin and heterochromatin

Samples from selected cell lines were collected and fixed as described in the previous section. Antibodies against the tri-methylated and acetylated forms of Lysine 9 of Histone H3 (Abcam PLC) were then employed to stain and analyze the cells.

### Results

To investigate possible correlations between cell radiosensitivity and induction of DSBs, 15 tumor cell lines were selected. In the tested 15 tumor cell lines, we determined radiosensitivity to killing using colony formation, induction of tDSBs using high temperature lysis (HTL) and of prDSBs using low temperature lysis (LTL). From these measurements the yields of tIDSBs can be estimated. Table 1 lists the cell lines employed, indicates their origins and shows their typical distribution throughout the cell cycle under the conditions used for experiments (see also Figure 1 for representative flow cytometry data). It is evident that under the conditions employed, all cell lines show similar distributions throughout the cell cycle. Furthermore, Table 1 also summarizes quantitative aspects of results presented and discussed below.

**Table 1**

| | | | | | |
|---|---|---|---|---|---|
| Alphabetical compilation of the cell lines tested, including information on their origin, cell cycle distribution, radiosensitivity to killing, as well as yields of tDSBs, prDSBs and tIDSBs. | | | | | |

| **Cell-line** | | **Origin** | **G1/S/G2, %** | | **Slope of HTL/Gy⁻¹** |
|---|---|---|---|---|---|
| A549 | | human lung adrenal carcinoma | 52/31/17 | 0.0101 | |
| A7 | | human glioma | 49/36/15 | 0.0110 | |
| Bogdahn 17 | | human glioma | 63/24/13 | 0.0106 | |
| C33A | | human cervical epithelial carcinoma | 57/31/12 | 0.0101 | |
| HCT116 | | human colorectal carcinoma | 38/34/28 | 0.0099 | |
| HeLa | | human cervical epithelial carcinoma | 48/39/13 | 0.0099 | |
| HT144 | | human melanoma | 50/35/15 | 0.0108 | |
| H460 | | human non-small lung carcinoma | 53/33/14 | 0.0099 | |
| H520 | | human non-small lung carcinoma | 46/31/23 | 0.0106 | |
| LnCap | | human prostate epithelial cancer | 56/24/20 | 0.0097 | |
| LN229 | | human glioma | 48/30/22 | 0.0104 | |
| PC3 | | human prostate epithelial cancer | 48/30/22 | 0.0101 | |
| SCC61 | | human head-neck squamous carcinoma | 43/35/22 | 0.0095 | |
| SQ20B | | human head-neck squamous carcinoma | 52/33/15 | 0.0092 | |
| U2OS | | human osteosarcoma | 39/41/20 | 0.0103 | |

| **Cell-line** | **Slope of LTL/Gy⁻¹** | **Slope of TLSL/Gy⁻¹** | **D37/Gy** | **D10/Gy** | **D1/Gy** |
|---|---|---|---|---|---|
| A549 | 0.0049 | 0.0052 | 4.1 | 5.9 | 9.6 |
| A7 | 0.0067 | 0.0043 | 2.0 | 3.8 | 6.4 |
| Bogdahn 17 | 0.0061 | 0.0045 | 2.8 | 5.3 | 8.2 |
| C33A | 0.0070 | 0.0041 | 0.9 | 3.1 | 5.2 |
| HCT116 | 0.0035 | 0.0054 | 3.9 | 6.1 | 10.0 |
| HeLa | 0.0046 | 0.0053 | 2.3 | 4.4 | 7.2 |
| HT144 | 0.0078 | 0.0030 | 0.9 | 1.8 | 3.3 |
| H460 | 0.0048 | 0.0051 | 2.3 | 5.0 | 7.7 |
| H520 | 0.0053 | 0.0054 | 2.2 | 3.9 | 7.3 |
| LnCap | 0.0044 | 0.0053 | 2.7 | 4.9 | 8.2 |
| LN229 | 0.0051 | 0.0053 | 3.0 | 5.6 | 8.0 |
| PC3 | 0.0054 | 0.0047 | 2.1 | 3.9 | 7.2 |
| SCC61 | 0.0044 | 0.0051 | 1.9 | 4.2 | 6.1 |
| SQ20B | 0.0034 | 0.0058 | 4.2 | 6.8 | 10.8 |
| U2OS | 0.0061 | 0.0042 | 1.9 | 3.7 | 7.1 |

Figures 2A, 2B and 2C depict the survival curves of the group; they document a wide spectrum of radiosensitivities as required by the aims of the present study.

Despite wide fluctuations in radiosensitivity to killing, the tested cell lines show relatively small fluctuations in the levels of tDSBs measured immediately after exposure to IR (Figure 3). This is better illustrated in Figure 4A, which integrates in a single plot the calculated lines for DSB yields. The results are in line with previous reports (see Introduction) suggesting that tDSB induction is only rarely a robust predictor of cell radiosensitivity to killing.

Notably, when yields of prDSBs are determined by LTL, marked differences are detected among cell lines (Figure 3C). This is again better illustrated in Figure 4B that depicts prDSB yields for all cell lines in a single graph.

The similarity in the induction of tDSBs among cell lines and the marked differences noted in the induction of prDSBs directly imply that the induction of tIDSBs will also be different in the different cell lines. We calculated therefore induction of tIDSBs by subtracting prDSBs from tDSBs and the results obtained are summarized in Figure 4C. As anticipated, marked differences in tIDSB induction are observed among tested cell lines.

Direct visual inspection and comparison between prDSB yields and cell radiosensitivity to killing reveals that radioresistant cells show low induction of prDSBs. The opposite is true for tIDSBs. This is quantitatively illustrated in Figure 5. Plotted in the figure for each cell line is the measured prDSB yields (slope of the dose response line, Table 1) as a function of radiation dose at which the survival of this cell line drops either to 37% (panel A) or to 10% (panel B). Results obtained by considering radiation doses corresponding to 1% cell survival are presented in Figure 6. The values of the corresponding parameters are included in Table 1.

Notably, comparison of prDSB yields with radiosensitivity to killing quantitatively confirms an excellent correlation. In this set of cell lines, HT144, the most radiosensitive cell line, shows a slope in DSB induction curves of 0.0078 Gy⁻¹, which is over twofold higher than the slope of SQ20B cells, 0.0034 Gy⁻¹, the most radioresistant cell line. Evidently, high yields of prDSBs render cells radiosensitive to killing and *vice-versa.*

For comparison, Figure 5C shows a plot similar to B but for tDSBs. It is evident that while a correlation between tDSB yields and radiation dose for 10% survival is observed, the available dynamic range and the statistical significance are by far not as robust as for prDSBs.

As outlined in the Introduction, analysis of γ-H2AX holds promise as predictor of radiosensitivity to killing. We wished to compare the predictive power of γ-H2AX, as a marker for DSB induction, with that of prDSBs-yields presented above. Therefore, we adapted a flow cytometry-based technology that allows analysis of integral γ-H₂AX signal in a range of radiation doses similar to that used in PFGE.

Typical histograms of γ-H₂AX signal obtained at different doses at 1 h postirradiation with the 15 cell lines under investigation are summarized in Figure 7. From such data, the mean γ-H2AX intensity can be estimated for each radiation dose. Among the different possible ways of presentation of this dose response, most informative proved the one that plots as a function of radiation dose the normalized intensity of γ-H2AX signal, after correction for DNA content. This value is calculated by dividing the mean γ-H2AX signal intensity obtained for a given radiation dose by the mean γ-H2AX signal intensity calculated for the 0 Gy sample.

Results of normalized γ-H2AX signal intensity as a function of radiation dose are summarized in Figure 8A for ten of the 15 cell lines utilized. Linear regression adequately fits the results obtained demonstrating that γ-H2AX signal saturation is not occurring in the range of doses examined.

The slopes of the dose response curves obtained show marked differences among cell lines, raising the potential of correlations with cell radiosensitivity to killing. Figure 8B shows the slopes of the γ-H2AX dose response of each cell line, plotted as a function of the radiation dose required for 10% cell survival. Notably, the results show no correlation whatsoever between DSB induction quantitated as normalized γ-H2AX signal and cell radiosensitivity to killing. We note that γ-H2AX generates signals reflecting tDSBs (29), and that γ-H2AX generation is subject to a complex physiological regulation, which may disconnect signal intensity from the number of DSBs present (27).

We inquired whether the low induction of prDSBs measured in radioresistant cell lines correlates with aspects of chromatin organization. Therefore, we screened ten of the 15 cell lines with known markers of chromatin organization. Trimethylation of Histone-H3-Lys9 (H3-3meK9) is a widely accepted marker of condensed, heterochromatic organization (34)(35). We used flow cytometry to estimate integral H3-3meK9 signal, as a measure of heterochromatin content, in our panel of cell lines. Raw measurements of this analysis are shown in Figure 9A. It is evident that quantitative differences exist among cell lines, indicated by fluctuations in mean signal intensity after correction for DNA content (Figure 9B). However, when the DNA content-corrected H3-3meK9 signal is correlated with cell radiosensitivity to killing (10% survival) trends are apparent, but without statistical significance (Figure 10).

As a complementary form of analysis we measured acetylation of the same Histone H3-Lys9, which is widely considered a measure of chromatin relaxation, i.e. of euchromatin (34, 35). We analyzed therefore again ten of the cell lines for H3-acK9 using flow cytometry. Raw measurements are summarized in Figure 11A and their quantification (mean of signal intensity after correction for DNA content) in Figure 11B. Plotting of the DNA content-corrected mean H3-acK9 signal against cell radiosensitivity to killing (10% survival; Figure 12) shows again trends, but no statistically significant correlation.

We conclude that the chosen parameters of chromatin organization fail to correlate with cell radiosensitivity to killing and that no significance should be placed in the observed trends.

### Discussion

The results provide, for the first time, evidence for an excellent correlation between prDSB-yields and cell radiosensitivity to killing and define a new parameter with strong predictive power. The results generate a basis for focusing on specific aspects of DSB induction for predicting radiosensitivity to killing at the expense of the more elaborate assays that are based on estimates of DSB repair capacity. While repair capacity certainly remains a key determinant of the cellular response to IR, our observations suggest that lethal events are generated with higher probability from prDSBs. We elaborate below that not only the predictive power of prDSB-yields is higher, but also their determination is far easier and more accurate than determination of repair capacity.

Defining surrogate predictors of cell radiosensitivity to killing is significant, as radiosensitivity of tumor cells is linked to tumor radiation response (10-12), and radiosensitivity of fibroblasts to late radiation effects (1-9) (see Introduction). Comparison with γ-H2AX- or tDSBs-yields demonstrates that under the conditions examined, the predictive power of prDSBs-yields for cell radiosensitivity to killing is superior.

The significance of our observations is further reinforced by the fact that determination of prDSB-yields using existing PFGE methods is straightforward and can be achieved with a high level of confidence. This is because the determination is based on an entire dose response curve with multiple dose points that is typically linear (Figure 3). Since prDSBs yields are reflected, in a first approximation, by the slope of the resulting line, they can be accurately determined by linear regression. In addition, the approach relies on results obtained at high doses of radiation generating PFGE-signals that can be accurately measured. Yet, it predicts radiosensitivity at 37% and 10% survival levels (Figure 5) that are achieved at doses well within the range of those routinely used in radiation oncology.

Previous work found relatively weak associations between tDSBs yields and radiosensitivity to killing (5, 10-12, 19) (see Introduction), an observation that is also supported by our results with tDSBs and γ-H2AX (Figures 5C and 8). Repair kinetics or residual DSBs, on the other hand, correlate with cell radiosensitivity to killing (6, 8, 9, 16, 23-26). Yet, accurate determination of the latter parameters is more demanding than measurement of prDSB-yields, as it requires maintenance of cells under conditions ensuring metabolic function equivalent to the *in-vivo* situation in order to maintain unchanged their repair potential. Even when this is achievable, very high radiation doses are required to obtain statistically significant differences in the number of unrepaired DSBs between cell lines with all assays that measure the physical presence of a DSB - e.g. PFGE (7-12). Use of γ-H₂AX based assays, however, ameliorates this concern (36).

Also the time point after radiation exposure at which residual DSB measurements are made is frequently debated, with different times after irradiation arbitrary chosen in different studies and actually showing different predictive power (19, 36). Measurement of prDSBs immediately after IR eliminates all these confounding factors and simplifies decisively the associated experimental protocol.

Importantly, this in-vitro method may be directly applied to biopsy material from an individual, obviating the tedious and time consuming step of establishing *in vitro* cultures. In this way, direct measurements may be possible using biopsy material, with the significant advantage of obtaining data actually reflecting the radiosensitivity of the tissue of origin (normal tissue or tumor), rather than that of cells selected by their ability to grow *in vitro.* There are intensive efforts at present along these lines in the field (36).

However, since biopsies may contain relatively few cells (10⁵ to 10⁶ cells for skin biopsies), the PFGE method may be employed after miniaturization that makes it feasible with fewer cells. Since key in the predictive power of the present assay is the selective measurement of prDSBs, methods may be applied providing this information on the basis of single-cell gel electrophoresis.

Why are tDSBs-yields or γ-H₂AX signal a weak predictor of radiosensitivity to killing, while prDSB-yield appears so strongly predictive? It may be that lethal lesions arise predominantly from prDSBs, while tIDSBs are shunted with higher probability to error-free processing. Our attempts to link prDSB or tIDSB induction to salient features of chromatin organization did not prove informative (Figures 9-12). Certainly more work is required to address and possibly clarify this important issue.

We define prDSB-yields as a novel parameter with strong predictive power towards cell radiosensitivity to killing. We further define tIDSB-yields as a novel parameter with strong predictive power towards cell radiosensitivity to killing. We show that these parameters can easily and highly accurately be determined. The approach defined here offers tantalizing new possibilities for the development of predictive assays with direct and wide clinical applicability.

### References

(1) Little JB, Nove J, Strong LC, Nichols WW. Survival of Human Diploid Skin Fibroblasts from Normal Individuals after X-irradiation. Int J Radiat Biol. 1988;54:899-910.
(2) Loeffler JS, Harris JR, Dahlberg WK, Little JB. In Vitro Radiosensitivity of Human Diploid Fibroblasts Derived from Women with Unusually Sensitive Clinical Responses to Definitive Radiation Therapy for Breast Cancer. Radiat Res. 1990;121 :227-31.
(3) Burnet NG, Nyman J, Turesson I, Wurm R, Yarnold JR, Peacock JH. The relationship between cellular radiation sensitivity and tissue response may provide the basis for individualising radiotherapy schedules. Radiother Oncol. 1994;33:228-38.
(4) Burnet NG, Nyman J, Turesson I, Wurm R, Yarnold JR, Peacock JH. Prediction of normal-tissue tolerance to radiotherapy from in-vitro cellular radiation sensitivity. Lancet. 1992;339:1570-1.
(5) Wurm R, Burnet NG, Duggal N, Yarnold JR, Peacock JH. Cellular radiosensitivity and dna damage in primary human fibroblasts. Int J Radiat Oncol Biol Phys. 1994;30:625-33.
(6) Kiltie AE, Orton CJ, Ryan AJ, Roberts SA, Marples B, Davidson SE, et al. A correlation between residual DNA double-strand breaks and clonogenic measurements of radiosensitivity in fibroblasts from preradiotherapy cervix cancer patients. Int J Radiat Oncol Biol Phys. 1997;39:1137-44.
(7) Sarkaria JN, Bush C, Eady JJ, Peacock JH, Steel GG, Yarnold JR. Comparison between Pulsed-Field Gel Electrophoresis and the Comet Assay as Predictive Assays for Radiosensitivity in Fibroblasts. Radiat Res. 1998;150:17-22.
(8) Dikomey E, Brammer I, Johansen J, Bentzen SM, Overgaard J. Relationship between DNA double-strand breaks, cell killing, and fibrosis studied in confluent skin fibroblasts derived from breast cancer patients. Int J Radiat Oncol Biol Phys. 2000;46:481-90.
(9) Kasten-Pisula U, Tastan H, Dikomey E. Huge differences in cellular radiosensitivity due to only very small variations in double-strand break repair capacity. Int J Radiat Biol. 2005;81:409-19.
(10) McMillan TJ, Cassoni AM, Edwards S, Holmes A, Peacock JH. The Relationship of DNA Double-strand Break Induction to Radiosensitivity in Human Tumour Cell Lines. Int J Radiat Biol. 1990;58:427-38.
(11) Ruiz de Almodovar JM, Nunez MI, McMillan TJ, Olea N, Mort C, Villalobos M, et al. Initial radiation-induced DNA damage in human tumour cell lines: a correlation with intrinsic cellular radiosensitivity. Br J Cancer. 1994;69:457-62.
(12) Woudstra C, Driessen C, Konings AWT, Kampinga HH. DNA damage induction and tumour cell radiosensitivity: PFGE and halo measurements. Int J Radiat Biol. 1998;73:495-502.
(13) Brock WA, Tucker SL, Geara FB, Turesson I, Wike J, Nyman J, et al. Fibroblast radiosensitivity versus acute and late normal skin responses in patients treated for breast cancer. Int J Radiat Oncol Biol Phys. 1995;32:1371-9.
(14) Geara FB, Peters LJ, Ang KK, Wike JL, Brock WA. Prospective comparison of in vitro normal cell radiosensitivity and normal tissue reactions in radiotherapy patients. Int J Radiat Biol. 1993;27:1173-9.
(15) Johansen J, Bentzen SM, Overgaard J, Overgaard M. Relationship between the in vitro radiosensitivity of skin fibroblasts and the expression of subcutaneous fibrosis, telangiectasia, and skin erythema after radiotherapy. Radiother Oncol. 1996;40:101-9.
(16) Russell NS, Arlett CF, Bartelink H, Begg AC. Use of fluorescence in situ hybridization to determine the relationship between chromosome aberrations and cell survival in eight human fibroblast strains. Int J Radiat Biol. 1995;68:185-96.
(17) Schipler A, Iliakis G. DNA double-strand-break complexity levels and their possible contributions to the probability for error-prone processing and repair pathway choice. Nucleic Acids Res. 2013;41:7589-605.
(18) Radford IR. The Dose-response for Low-LET Radiation-induced DNA Double-strand Breakage: Methods of Measurement and Implications for Radiation Action Models. Int J Radiat Biol. 1988;54:1-11.
(19) El-Awady RA, Dikomey E, Dahm-Daphi J. Radiosensitivity of human tumour cells is correlated with the induction but not with the repair of DNA double-strand breaks. Br J Cancer. 2003;89:593-601.
(20) Okayasu R, Iliakis G. Linear DNA elution dose response curves obtained in CHO cells with non-unwinding filter elution after appropriate selection of the lysis conditions. Int J Radiat Biol. 1989;55:569-81.
(21) Okayasu R, Iliakis G. The shape of DNA elution dose response curves under non-denaturing conditions:. Int J Radiat Biol. 1992;61:455-63.
(22) Dahm-Daphi J, El-Awady RA, Dikomey E. Reply: Tumour and normal cells differ in the induction and repair of DNA double-strand breaks. Br J Cancer. 2004;90:556-.
(23) Koch U, Höhne K, von Neubeck C, Thames HD, Yaromina A, Dahm-Daphi J, et al. Residual γH2AX foci predict local tumour control after radiotherapy. Radiother Oncol. 2013;108:434-9.
(24) Menegakis A, Yaromina A, Eicheler W, Dorfler A, Beuthien-Baumann B, Thames HD, et al. Prediction of clonogenic cell survival curves based on the number of residual DNA double strand breaks measured by gH2AX staining. Int J Radiat Biol. 2009;85:1032-41.
(25) Banath J, Klokov D, MacPhail S, Banuelos C, Olive P. Residual gammaH2AX foci as an indication of lethal DNA lesions. BMC Cancer. 2010;10:4.
(26) Olive PL, Banath JP, Keyes M. Residual g H2AX after irradiation of human lymphocytes and monocytes in vitro and its relation to late effects after prostate brachytherapy. Radiother Oncol. 2008;86:336-46.
(27) Kinner A, Wu W, Staudt C, Iliakis G. g-H2AX in recognition and signaling of DNA double-strand breaks in the context of chromatin. Nucleic Acids Res. 2008;36:5678-94.
(28) Singh SK, Bencsik-Theilen A, Mladenov E, Jakob B, Taucher-Scholz G, Iliakis G. Reduced contribution of thermally labile sugar lesions to DNA double strand break formation after exposure to heavy ions. Radiat Oncol. 2013;8:77.
(29) Singh SK, Wang M, Staudt C, Iliakis G. Post-irradiation chemical processing of DNA damage generates double-strand breaks in cells already engaged in repair. Nucleic Acids Res. 2011;39:8416-29.
(30) Singh SK, Wu W, Stuschke M, Bockisch A, Iliakis G. Reduced Contribution of Thermally-Labile Sugar Lesions to DNA Double-Strand Break Formation after Exposure to Neutrons. Radiat Res. 2012;178:581-90.
(31) Singh SK, Wu W, Wu W, Wang M, Iliakis G. Extensive Repair of DNA Double-Strand Breaks in Cells Deficient in the DNA-PK Dependent Pathway of NHEJ after Exclusion of Heat-Labile Sites. Radiat Res. 2009;172:152-64.
(32) Stenerlow B, Karlsson KH, Cooper B, Rydberg B. Measurement of prompt DNA double-strand breaks in mammalian cells without including heat-labile sites: Results for cells deficient in nonhomologous end joining. Radiat Res. 2003;159:502-10.
(33) Rydberg B. Radiation-induced heat-labile sites that convert into DNA double-strand breaks. Radiat Res. 2000;153:805-12.
(34) Kakarougkas A, Ismail A, Klement K, Goodarzi AA, Conrad S, Freire R, et al. Opposing roles for 53BP1 during homologous recombination. Nucleic Acids Res. 2013;41:9719-31.
(35) Grewal SIS, Jia S. Heterochromatin revisited. Nat Rev Genet. 2007;8:35-46.
(36) Menegakis A, von Neubeck C, Yaromina A, Thames H, Hering S, Hennenlotter J, et al. γH2AX assay in ex vivo irradiated tumour specimens: A novel method to determine tumour radiation sensitivity in patient-derived material. Radiother Oncol. 2015;in press.
(37) Iliakis GE, Cicilioni O, Metzger L. Measurement of DNA double-strand breaks in CHO cells at various stages of the cell cycle using pulsed field gel electrophoresis: calibration by means of 1251 decay. Int J Radiat Biol. 1991 Feb;59(2):343-57.
(38) DiBiase SJ, Zeng ZC, Chen R, Hyslop T, Curran WJ Jr, Iliakis G. DNA-dependent protein kinase stimulates an independently active, nonhomologous, end-joining apparatus. Cancer Res. 2000 Mar 1;60(5):1245-53.

## Claims

1. An in vitro method for predicting the degree of radiosensitivity of a cell, comprising
(a) irradiating a cell,
(b) determining the number of prompt double-strand breaks (prDSBs) in the cell of step (a), and
(c) using the number of prDSBs determined in step (b) to predict the degree of radiosensitivity of said cell.

2. An in vitro method for predicting the degree of radiosensitivity of a cell, comprising
(a) irradiating a cell,
(b) determining the number of thermally labile sugar lesion-dependent double-strand breaks (tIDSBs)) in the cell of step (a), and
(c) using the number of tIDSBs determined in step (b) to predict the degree of radiosensitivity of said cell.

3. The method of claim 2, wherein the number of tIDSBs is determined by subtracting the number of prDSBs from the number of total double-strand breaks (tDSBs).

4. The method of any one of claims 1 to 3, wherein the cell is a diseased cell, preferably a tumor cell, more preferably of epithelial origin, of mesenchymal origin, of hematopoietic origin, or of neuro-ectodermal origin, still more preferably, the cell is selected from a breast adenocarcinoma, sweat gland adenocarcinoma, salivary gland adenocarcinoma, skin squamous cell carcinoma, adenocarcinoma of the thyroid, lung, stomach, liver, pancreas, small intestine, colon, or prostate, transitional cell carcinoma of the bladder; adenocarcinoma of the kidney, testis or endometrium, fibrosarcoma, liposarcoma, osteosarcoma, chondrosarcoma, leiomyosarcoma, hemangiosarcoma, lymphoma, leukemia, astrocytoma, retinoblastoma, oligodendroglioma, schwannoma, melanoma, head and neck cancer, ovarian cancer, adenoid carcinoma, basal cell carcinoma, epidermoid carcinoma, meningioma, neurofibroma, glioblastoma, ependymoma, medulloblastoma, neuroblastoma, hepatoma, mesothelioma, brain cancer such as glioblastoma multiforme, hepatoma, lymphoma, myeloma, neuroblastoma, sarcoma, stomach cancer, thyroid cancer, non-melanoma skin cancer, non-small cell lung cancer, cervical cancer, or anal cancer, or
preferably from the thyroid gland in case of the disease of Basedow or hyperthyroidism, from the pituitary gland in case of pituitary adenoma, from the meninges in case of a meningioma, from the skin with a non-cancerous skin disorder, particularly rosacea, poikiloderma of Civatte, angioma, telangiectasias, or psoriasis, or from the ankle in case of talalgia.

5. The method of any one of claims 1 to 4, wherein the degree of radiosensitivity of said cell is predicted with respect to a reference.

6. The method of claim 5, wherein the reference is a diseased individual, diseased tissue or diseased cell or a plurality of diseased individuals, diseased tissues or diseased cells, wherein the disease may be a tumor or a disease, as defined in claim 4, preferably wherein the reference is a tumor cell or a plurality of tumor cells, or wherein the reference is a normal individual, normal tissue or normal cell or a plurality of normal individuals, normal tissues or normal cells.

7. The method of claim 6, wherein the disease, preferably the tumor, is known to be treatable by radiotherapy.

8. The method of claim 7, wherein the radiation dose for treating the disease of the reference, preferably tumor, is known.

9. The method of any one of claims 4 to 8 for determining the radiation dose for treating a diseased cell, preferably a tumor cell, in an individual, the method further comprising
d) comparing the radiosensitivity of the diseased cell, preferably the tumor cell, with the radiosensitivity of the reference, and
e) determining the radiation dose for treating the diseased cell, preferably the tumor cell.

10. The method of any one of claims 1 to 3, wherein the cell is a normal cell, preferably an epithelial cell, such as a keratinocyte or a lens epithelial cell, a melanocyte, a cardiac myocyte, a chrondrocyte, an endothelial cell, a fibroblast, an osteoblast, a preadipocyte, a skeletal muscle cell, a smooth muscle cell, or a lymphocyte.

11. The method of claim 10, wherein the degree of radiosensitivity of said cell is predicted with respect to a reference.

12. The method of any one of claims 4 to 11, wherein the reference is a normal individual, normal tissue or normal cell or a plurality of normal individuals, normal tissues or normal cells.

13. The method of any one of claims 1 to 12, wherein the cell is irradiated with ionizing radiation.

14. The method of any one of claims 1 to 13, wherein the number of prDSBs or tIDSBs is determined using pulsed-field gel electrophoresis, preferably asymmetric field inversion gel electrophoresis, or the "Comet" assay.

15. The method of any one of claims 1 to 14, wherein the number of prDSBs or tIDSBs is determined by the fraction of DNA released (FDR).

## Patentansprüche

1. In vitro-Verfahren zum Vorhersagen des Grades der Strahlenempfindlichkeit einer Zelle, umfassend
(a) Bestrahlen einer Zelle,
(b) Bestimmen der Anzahl von prompten Doppelstrangbrüchen (prDSBs) in der Zelle von Schritt (a), und
(c) Verwenden der in Schritt (b) bestimmten Anzahl von prDSBs zum Vorhersagen des Grades der Strahlenempfindlichkeit der Zelle.

2. In vitro-Verfahren zum Vorhersagen des Grades der Strahlenempfindlichkeit einer Zelle, umfassend
(a) Bestrahlen einer Zelle,
(b) Bestimmen der Anzahl von thermisch labilen, zuckerläsionsabhängigen Doppelstrangbrüchen (tIDSBs)) in der Zelle von Schritt (a), und
(c) Verwenden der in Schritt (b) bestimmten Anzahl von tIDSBs zum Vorhersagen des Grades der Strahlenempfindlichkeit der Zelle.

3. Verfahren nach Anspruch 2, wobei die Anzahl der tIDSBs durch Subtrahieren der Anzahl von prDSBs von der Anzahl von gesamten Doppelstrangbrüchen (tDSBs) bestimmt wird.

4. Verfahren nach einem beliebigen der Ansprüche 1 bis 3, wobei die Zelle eine kranke Zelle ist, vorzugsweise eine Tumorzelle, stärker bevorzugt epithelialen Ursprungs, mesenchymalen Ursprungs, hämatopoetischen Ursprungs oder neuroektodermalen Ursprungs, noch stärker bevorzugt ist die Zelle ausgewählt aus einem Brust-Adenokarzinom, Schweißdrüsen-Adenokarzinom, Speicheldrüsen-Adenokarzinom, Haut-Plattenepithelkarzinom, Adenokarzinom der Schilddrüse, Lunge, des Magens, der Leber, Bauchspeicheldrüse, des Dünndarms, des Dickdarms oder der Prostata, Übergangszellkarzinom der Blase; Adenokarzinom der Niere, des Hodens oder des Endometriums, Fibrosarkom, Liposarkom, Osteosarkom, Chondrosarkom, Leiomyosarkom, Hämangiosarkom, Lymphom, Leukämie, Astrozytom, Retinoblastom, Oligodendrogliom, Schwannom, Melanom, Kopf- und Halskrebs, Eierstockkrebs, Adenoidkarzinom, Basalzellkarzinom, Epidermoidkarzinom, Meningiom, Neurofibrom, Glioblastom, Ependymom, Medulloblastom, Neuroblastom, Hepatom, Mesotheliom, Hirnkrebs wie Glioblastoma multiforme, Hepatom, Lymphom, Myelom, Neuroblastom, Sarkom, Magenkrebs, Schilddrüsenkrebs, nicht-melanomaler Hautkrebs, nicht-kleinzelliger Lungenkrebs, Gebärmutterhalskrebs oder Analkrebs, oder
vorzugsweise von der Schilddrüse im Fall der Basedow-Krankheit oder von Schilddrüsenüberfunktion, von der Hypophyse im Fall von Hypophysenadenom, von den Hirnhäuten im Fall eines Meningeoms, von der Haut mit einer nicht kanzerösen Hauterkrankung, insbesondere Rosazea, Civatte-Poikilodermie, Angiom, Teleangiektasien oder Psoriasis, oder von dem Knöchel im Fall von Talalgie.

5. Verfahren nach einem beliebigen der Ansprüche 1 bis 4, wobei der Grad der Strahlenempfindlichkeit der Zelle in Bezug auf eine Referenz vorhergesagt wird.

6. Verfahren nach Anspruch 5, wobei die Referenz ein erkranktes Individuum, erkranktes Gewebe oder eine erkrankte Zelle oder eine Vielzahl von erkrankten Individuen, erkrankten Geweben oder erkrankten Zellen ist, wobei die Krankheit ein Tumor oder eine Krankheit, wie in Anspruch 4 definiert, sein kann, vorzugsweise wobei die Referenz eine Tumorzelle oder eine Vielzahl von Tumorzellen ist, oder wobei die Referenz ein normales Individuum, normales Gewebe oder eine normale Zelle oder eine Vielzahl von normalen Individuen, normalen Geweben oder normalen Zellen ist.

7. Verfahren nach Anspruch 6, wobei die Krankheit, vorzugsweise der Tumor, bekanntermaßen durch Strahlentherapie behandelbar ist.

8. Verfahren nach Anspruch 7, wobei die Strahlendosis zum Behandeln der Krankheit der Referenz, vorzugsweise des Tumors, bekannt ist.

9. Verfahren nach einem beliebigen der Ansprüche 4 bis 8 zum Bestimmen der Strahlungsdosis zum Behandeln einer erkrankten Zelle, vorzugsweise einer Tumorzelle, in einem Individuum, wobei das Verfahren ferner umfasst
d) Vergleichen der Strahlenempfindlichkeit der erkrankten Zelle, vorzugsweise der Tumorzelle, mit der Strahlenempfindlichkeit der Referenz, und
e) Bestimmen der Strahlendosis zum Behandeln der erkrankten Zelle, vorzugsweise der Tumorzelle.

10. Verfahren nach einem beliebigen der Ansprüche 1 bis 3, wobei die Zelle eine normale Zelle, vorzugsweise eine Epithelzelle wie ein Keratinozyt- oder eine Linsenepithelzelle, ein Melanozyt, eine Herzmuskelzelle, ein Chondrozyt, eine Endothelzelle, ein Fibroblast, ein Osteoblast, ein Präadipozyt, eine Skelettmuskelzelle, glatte Muskelzelle oder ein Lymphozyt ist.

11. Verfahren nach Anspruch 10, wobei der Grad der Strahlenempfindlichkeit der Zelle in Bezug auf eine Referenz vorhergesagt wird.

12. Verfahren nach einem beliebigen der Ansprüche 4 bis 11, wobei die Referenz ein normales Individuum, normales Gewebe oder eine normale Zelle oder eine Vielzahl von normalen Individuen, normalen Geweben oder normalen Zellen ist.

13. Verfahren nach einem beliebigen der Ansprüche 1 bis 12, wobei die Zelle mit ionisierender Strahlung bestrahlt wird.

14. Verfahren nach einem beliebigen der Ansprüche 1 bis 13, wobei die Anzahl von prDSBs oder tIDSBs unter Verwenden von Puls-Feld-Gelelektrophorese, vorzugsweise von asymmetrischer Feld-Inversions-Gelelektrophorese, oder des "Comet"-Assays bestimmt wird.

15. Verfahren nach einem beliebigen der Ansprüche 1 bis 14, wobei die Anzahl der prDSBs oder tIDSBs durch die Fraktion von freigesetzter DNA (FDR) bestimmt wird.

## Revendications

1. Procédé in vitro pour prédire le degré de radiosensibilité d'une cellule, comprenant les étapes consistant en
(a) l'irradiation d'une cellule,
(b) la détermination du nombre de cassures rapides de doubles brins (prDSB) dans la cellule de l'étape (a), et
(c) l'utilisation du nombre de prDSB, déterminé dans l'étape (b), pour prédire le degré de radiosensibilité de ladite cellule.

2. Procédé in vitro pour prédire le degré de radiosensibilité d'une cellule, comprenant les étapes consistant en
(a) l'irradiation d'une cellule,
(b) la détermination du nombre de cassures de doubles brins dépendantes de lésions de sucres labiles thermiquement (tlDSB) dans la cellule de l'étape (a), et
(c) l'utilisation du nombre de tlDSB, déterminé dans l'étape (b), pour prédire le degré de radiosensibilité de ladite cellule.

3. Procédé selon la revendication 2, dans lequel le nombre de tlDSB est déterminé par soustraction du nombre de prDSB du nombre de cassures totales de doubles brins (tDSB) .

4. Procédé selon l'une quelconque des revendications 1 à 3, dans lequel la cellule est une cellule malade, de préférence une cellule tumorale, plus préférablement d'origine épithéliale, d'origine mésenchymateuse, d'origine hématopoïétique, ou d'origine neuro-ectodermique, encore plus préférablement la cellule est choisie parmi un adénocarcinome du sein, un adénocarcinome des glandes sudoripares, un adénocarcinome des glandes salivaires, un carcinome cutané à cellules squameuses, un adénocarcinome de la thyroïde, du poumon, de l'estomac, du foie, du pancréas, de l'intestin grêle, du côlon, ou de la prostate, un carcinome de la vessie à cellules de transition ; un adénocarcinome du rein, des testicules ou de l'endomètre, un fibrosarcome, un liposarcome, un ostéosarcome, un chondrosarcome, un leiomyosarcome, un hémangiosarcome, un lymphome, une leucémie, un astrocytome, un rétinoblastome, un oligodendrogliome, un schwannome, un mélanome, un cancer de la tête et du cou, un cancer ovarien, un carcinome adénoïde, un carcinome à cellules basales, un carcinome épidermoïde, un méningiome, un neurofibrome, un glioblastome, un épendymome, un médulloblastome, un neuroblastome, un hépatome, un mésothéliome, un cancer du cerveau tel qu'un glioblastome multiforme, un hépatome, un lymphome, un myélome, un neuroblastome, un sarcome, un cancer de l'estomac, un cancer de la thyroïde, un cancer de la peau non-mélanome, un cancer du poumon non à petites cellules, un cancer du col utérin, ou un cancer anal, ou
de préférence à partir de la glande thyroïde dans le cas de la maladie de Basedow ou d'une hyperthyroïdie, de l'hypophyse dans le cas d'un adénome hypophysaire, des méninges dans le cas d'un méningiome, de la peau souffrant d'un trouble cutané non cancéreux, en particulier d'une rosacée, d'une poïkilodermie de Civatte, d'un angiome, d'une télangiectasie, ou d'un psoriasis, ou de la hanche dans le cas d'une talalgie.

5. Procédé selon l'une quelconque des revendications 1 à 4, dans lequel le degré de radiosensibilité de ladite cellule est prédit par rapport à une référence.

6. Procédé selon la revendication 5, dans lequel la référence est un individu malade, un tissu malade ou une cellule malade ou une pluralité d'individus malades, de tissus malades ou de cellules malades, où la maladie peut être une tumeur ou une maladie, telle que définie dans la revendication 4, de préférence dans lequel la référence est une cellule tumorale ou une pluralité de cellules tumorales, ou dans lequel la référence est un individu normal, un tissu normal ou une cellule normale, ou une pluralité d'individus normaux, de tissus normaux ou de cellules normales.

7. Procédé selon la revendication 6, dans lequel la maladie, de préférence la tumeur, est connue pour pouvoir être traitée par radiothérapie.

8. Procédé selon la revendication 7, dans lequel la dose d'irradiation pour traiter la maladie de la référence, de préférence la tumeur, est connue.

9. Procédé selon l'une quelconque des revendications 4 à 8 pour déterminer la dose d'irradiation pour traiter une cellule malade, de préférence une cellule tumorale, chez un individu, le procédé comprenant en outre les étapes consistant en
d) la comparaison de la radiosensibilité de la cellule malade, de préférence de la cellule tumorale, avec la radiosensibilité de la référence, et
e) la détermination de la dose d'irradiation pour traiter la cellule malade, de préférence la cellule tumorale.

10. Procédé selon l'une quelconque des revendications 1 à 3, dans lequel la cellule est une cellule normale, de préférence une cellule épithéliale, telle qu'un kératinocyte ou une cellule épithéliale de cristallin, un mélanocyte, un myocyte cardiaque, un chondrocyte, une cellule endothéliale, un fibroblaste, un ostéoblaste, un pré-adipocyte, une cellule musculaire squelettique, une cellule musculaire lisse, ou un lymphocyte.

11. Procédé selon la revendication 10, dans lequel le degré de radiosensibilité de ladite cellule est prédit par rapport à une référence.

12. Procédé selon l'une quelconque des revendications 4 à 11, dans lequel la référence est un individu normal, un tissu normal ou une cellule normale ou une pluralité d'individus normaux, de tissus normaux ou de cellules normales.

13. Procédé selon l'une quelconque des revendications 1 à 12, dans lequel la cellule est irradiée avec un rayonnement ionisant.

14. Procédé selon l'une quelconque des revendications 1 à 13, dans lequel le nombre de prDSB ou de tlDSB est déterminé par utilisation d'une électrophorèse sur gel en champs pulsés, de préférence d'une électrophorèse sur gel en champs alternatifs asymétriques, ou d'un dosage "Comet".

15. Procédé selon l'une quelconque des revendications 1 à 14, dans lequel le nombre de prDSB ou de tlDSB est déterminé par la fraction de l'ADN libéré (FDR).
